# EUROPEAN PATENT APPLICATION

(11) **EP 4 071 165 A1**
(43) Date of publication of application: **12.10.2022**
(21) Application number: 21382283.6
(22) Date of filing: 05.04.2021
(51) Int. Cl.: C07K 14/47, A61P 25/00, A61P 25/28, G01N 33/68

(54) **A NEW NON-AGGREGATIVE SPLICING ISOFORM OF HUMAN TAU IS DECREASED IN ALZHEIMER S DISEASE**

(71) Applicant: Consejo Superior de Investigaciones Científicas (CSIC), 28006 Madrid (ES); Universidad Autónoma de Madrid (UAM), 28770 Madrid (ES)
(72) Inventor: AVILA DE GRADO, Jesús, 28049 Madrid (ES); LUCAS LOZANO, José Javier, 28049 Madrid (ES); CUADROS CATALÁN, Raquel, 28049 Madrid (ES); GARCÍA GARCÍA, Esther, 28049 Madrid (ES); GARCÍA ESCUDERO, Vega, 28049 Madrid (ES); HERNÁNDEZ PÉREZ, Félix, 28049 Madrid (ES); RUIZ GABARRE, Daniel, 28049 Madrid (ES)
(74) Representative: Hoffmann Eitle

(57) **Abstract**

The present invention refers to a composition of matter comprising at least one peptide inhibitor (from hereinafter "peptide inhibitor/s of the invention") that reduces or prevents amyloid beta (Ab) and/or tau aggregation and toxicity, preferably of already-aggregated species, wherein the at least one peptide inhibitor comprises or consists of an amino acid sequence WVGCCPW (SEQ ID NO 1), KKVKGVGWVGCCPWVYGH (SEQ ID NO 2), GVGWVG (SEQ ID NO 3), GWVGCCPW (SEQ ID NO 4), VGWVGCCPW (SEQ ID NO 5), GVGWVGCCPW (SEQ ID NO 6), KGVGWVGCCPW (SEQ ID NO 7), VKGVGWVGCCPW (SEQ ID NO 8), WVGCCPWV (SEQ ID NO 9), GWVGCCPWV (SEQ ID NO 10), WVGCCPWVY (SEQ ID NO 11), WVGCCPWVYG (SEQ ID NO 12) or WVGCCPWVYGH (SEQ ID NO: 13) or any combination thereof.

## Description

### Technical field

The invention relates to compositions and methods useful in inhibiting aggregation of Tau and amyloid b proteins.

### Background of the invention

Tau is a microtubule-associated protein (MAP) that acts as a neurite microtubule stabilizer [9, 61]. Tau overexpression, especially in its modified forms (i.e. phosphorylated, aggregated or truncated) may result in a toxic gain of function [5]. Human Tau protein isoforms are expressed from a unique gene (*MAPT*) located at chromosome 17 [30, 42] and has at least 16 exons [2, 3]. Exons 1, 4, 5, 7, 9, 11, 12 and 13 are constitutive while the others are subjected to alternative splicing [2, 60]. Importantly, over 90% of human genes allegedly undergo alternative splicing [59], although recent evidence point out that this may not be the main mechanism to explain proteome complexity [55]. However, there is robust evidence for certain genes being subjected to alternative splicing, yielding well-studied spliced variants, being *MAPT* one of the most relevant [1, 55]. Noteworthy, aberrant splicing has been associated with numerous diseases, including Alzheimer's disease and other age-related disorders [51]. In accordance with that, mutations altering alternative splicing of exon 10 of *MAPT* cause some tauopathies, including FTDP-17 [16, 25, 32], and may be associated with others such as Huntington's disease, whose patients display a four-repeat tauopathy with nuclear rods [19].

Truncated Tau proteins lead to a toxic gain of function, promoting abnormal microtubule assembly and inducing aggregation, being a key feature of Alzheimer's disease, especially in the sporadic form [43, 65].

Tau truncation can take place at N-terminal or C-terminal regions [6, 22, 41, 44, 46, 48, 52, 63, 64]. Recently, a truncated toxic Tau fragment raised upon asparagine endopeptidase (AEP) cleavage, has been reported in mouse and elderly and Alzheimer's disease human brains [63]. This toxic fragment contains residues 1-368 of the human Tau molecule and the last residue (asparagine 368) coincides with the end of exon 12.

In the present invention, we present evidence of a new, human-specific truncated form of Tau similar to Tau 1-368 generated by intron 12 retention in human neuroblastoma cell lines and brain. An analogous mechanism of truncation has been described for a toxic huntingtin fragment [52]. **This intron-retaining RNA species would generate a truncated Tau protein similar to the one resulting from AEP cleavage, but followed by 18 extra amino acids, which dramatically reduces its ability to aggregate.**

Strikingly, the resulting Tau truncated isoform is reduced in the later stages of Alzheimer's disease patients' brain, in contrast to increasing total Tau levels. Despite the similarity of its sequence to that of the AEP truncated isoform, this new Tau isoform is able to bind to microtubules and is less prone to aggregate, suggesting a beneficial role in the pathology.

### Brief description of the figures

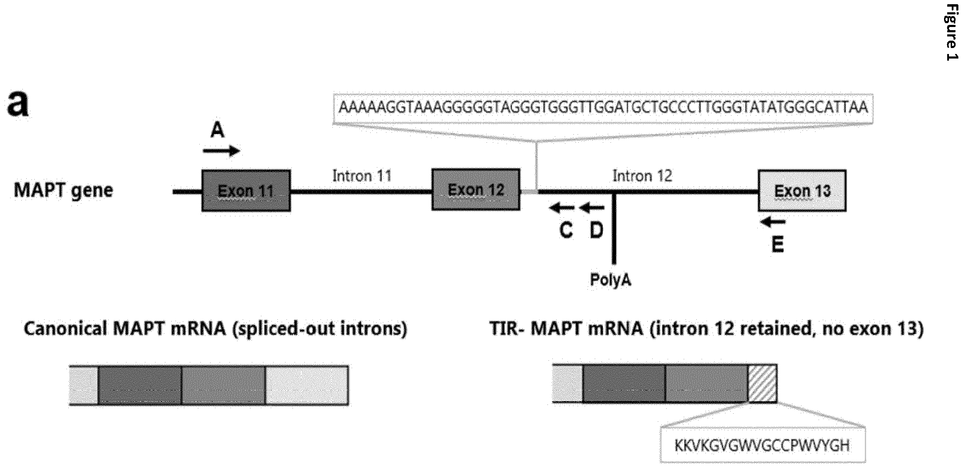

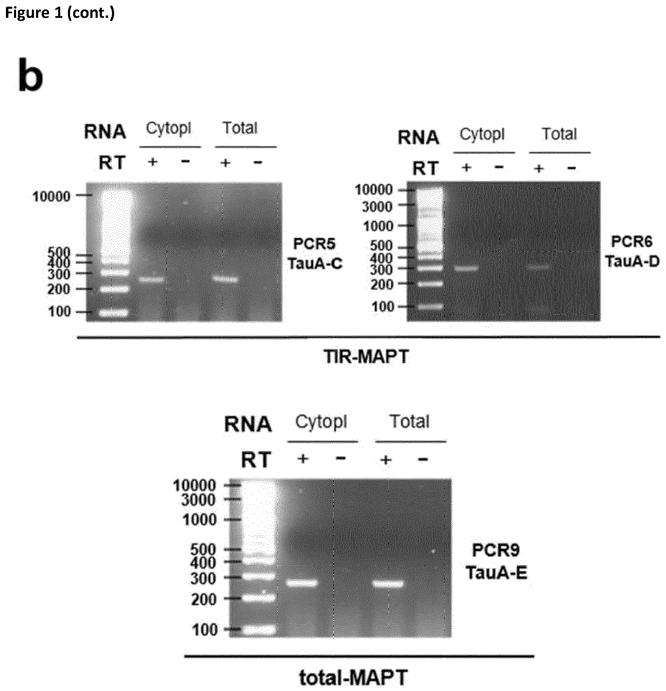

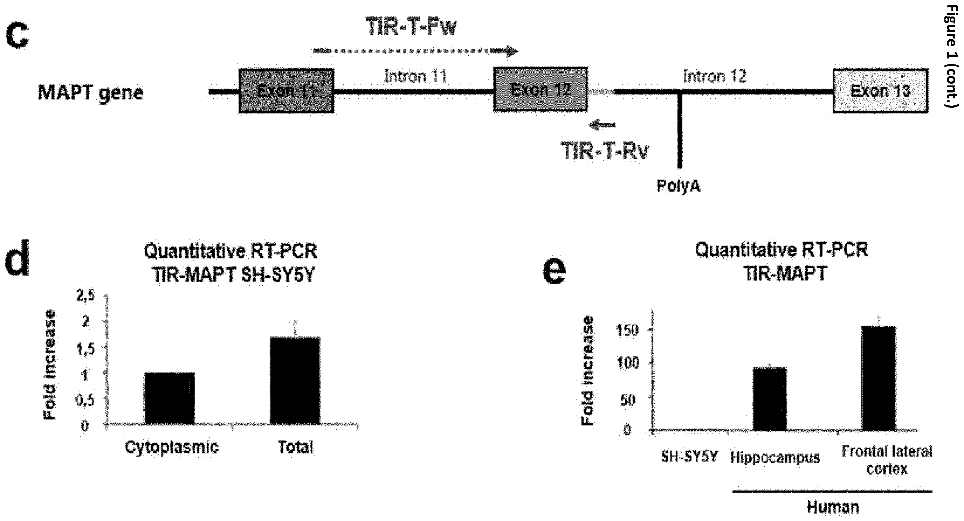

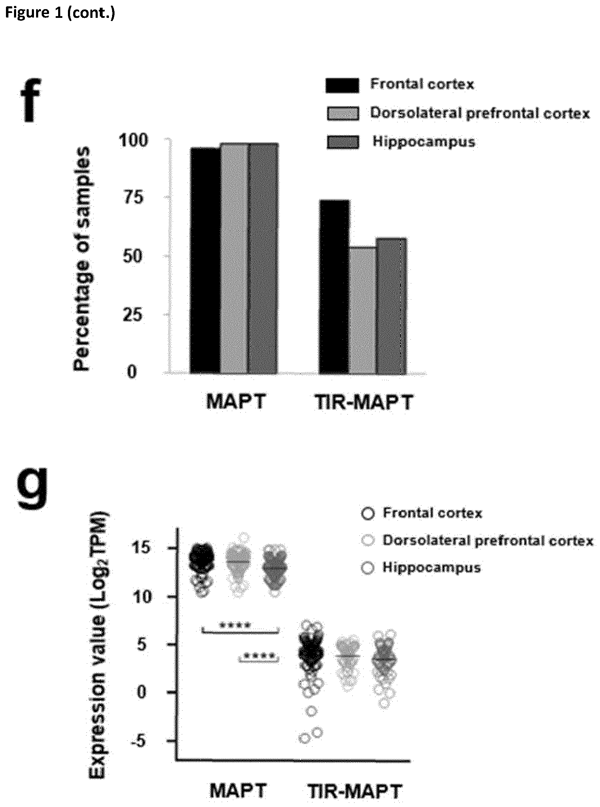

**Figure 1** ***TIR-MAPT* RNA expression. a.** Schematic representation of the *MAPT* gene and *MAPT* and *TIR-MAPT* mRNAs generated from it. A, C, D and E represent the hybridization sites of the primers designed for semi-quantitative PCR employed for the PCRs in **b (Table 1).** The fragment of intron 12 that would remain upon retention is represented with colored stripes. **b.** Representative images of agarose gels showing of semi-quantitative PCR results using total or cytoplasmic-enriched RNA of SH-SY5Y cells. Results showed the existence of RNA species from exon 11 to intron 12 where intron 11 was spliced out (PCR 5 and 6). Controls of the addition (RT+) or no addition of retrotranscriptase (RT-) were included. PCR 9 show *MAPT* in which both intron 11 and 12 are spliced out. Detailed information of all semi-quantitative PCR combinations and amplicon sizes is provided in **Table 4. c.** Schematic representation of the *MAPT* gene including the hybridization sites of the oligos used for quantitative PCR. **d.** *TIR-MAPT* RNA levels by qPCR in cytoplasmic enriched fraction or whole extracts (total) of SH-SY5Y cells. **e.** Comparison of *TIR-MAPT* level in SH-SY5Y cells and hippocampus and frontal lateral cortex of human brain. Graphs show means and SE of technical triplicates. **f.** Percentage of brain samples having expression of *MAPT* or *TIR-MAPT* genes. Data is shown for 3 different regions within brains (see **Table 2). g.** Scatter dot blot of expression values of *MAPT* and *TIR-MAPT* genes in brain regions having TPM (Transcripts Per Kilobase Million) > 0. Number of samples: cortex=122; frontal cortex=113; hippocampus=98. Graphs represent mean and SEM. p-values were calculated using a T' test, **** p-value ≤ 0.0001

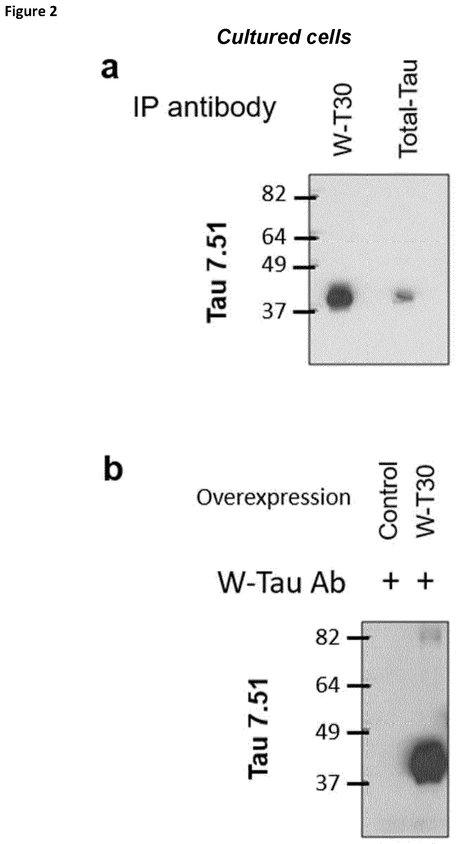

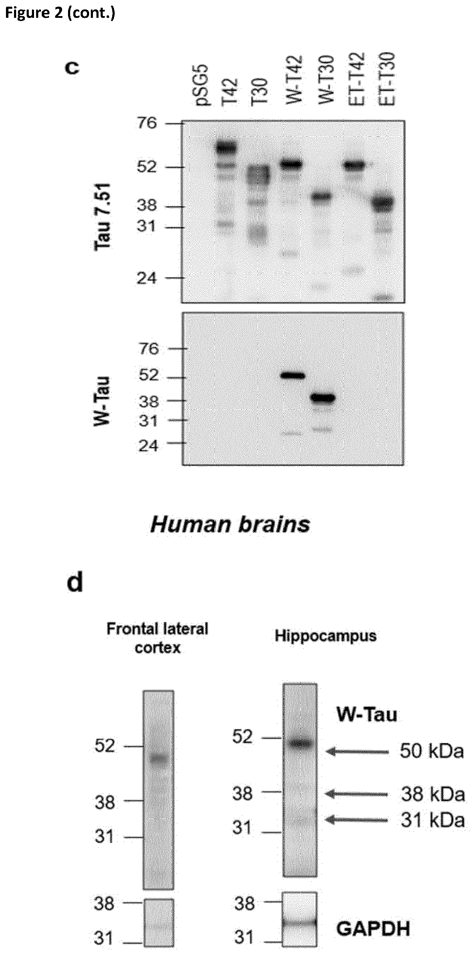

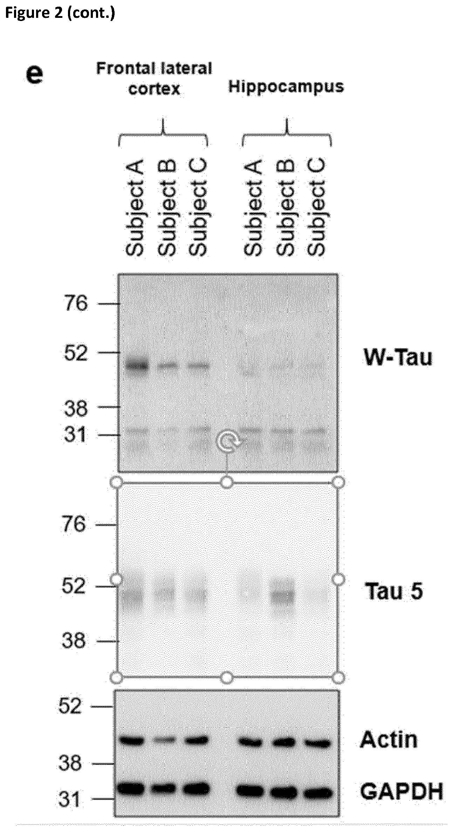

**Figure 2** **W-Tau antibody validation and protein expression in human brain. a.** Immunoprecipitation assay of HEK239T cells overexpressing W-T30 using W-Tau (Abyntek) or total Tau (NOVUSBIO, NB100-82247) antibodies and Western blot detection of immunoprecipitated Tau using 7.51 antibody. **b.** Immunoprecipitation of the same cells and control untransfected cells using W-Tau antibody and detection of immunoprecipitated Tau using total Tau (Tau 7.51) antibody. **c.** HEK293T cells were transfected with eukaryotic expression vectors empty or encoding different *MAPT* isoforms (T42, T30, W-T42, W-T30, ET-42 and ET-T30). Representative Western Blot for Tau 7.51 and W-Tau antibodies proving W-Tau specificity. **d.** Western blot detection of W-Tau in frontal lateral cortex and hippocampus of one human subject that show bands at 50 kDa (W-T42), 38 KDa (W-T30), and 31 KDa (W-Tau truncated). **e.** Representative Western blot of frontal lateral cortex and hippocampus samples of the same 3 human subjects (A, B and C) showing the presence of W-Tau and total-Tau (Tau5).

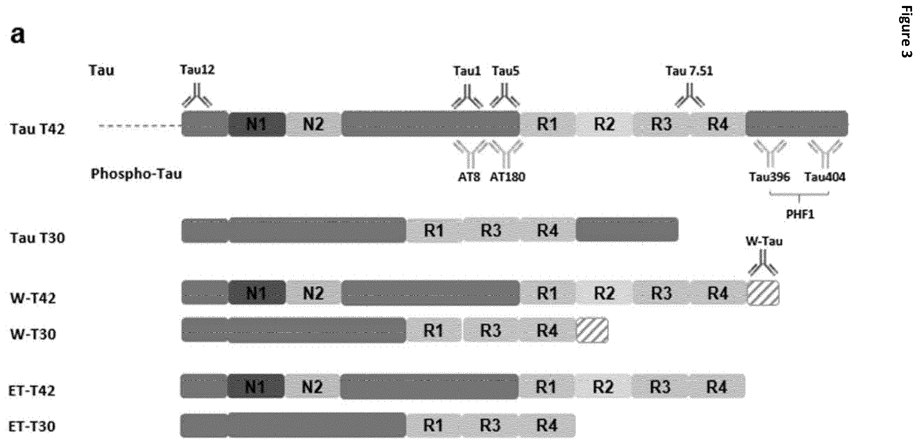

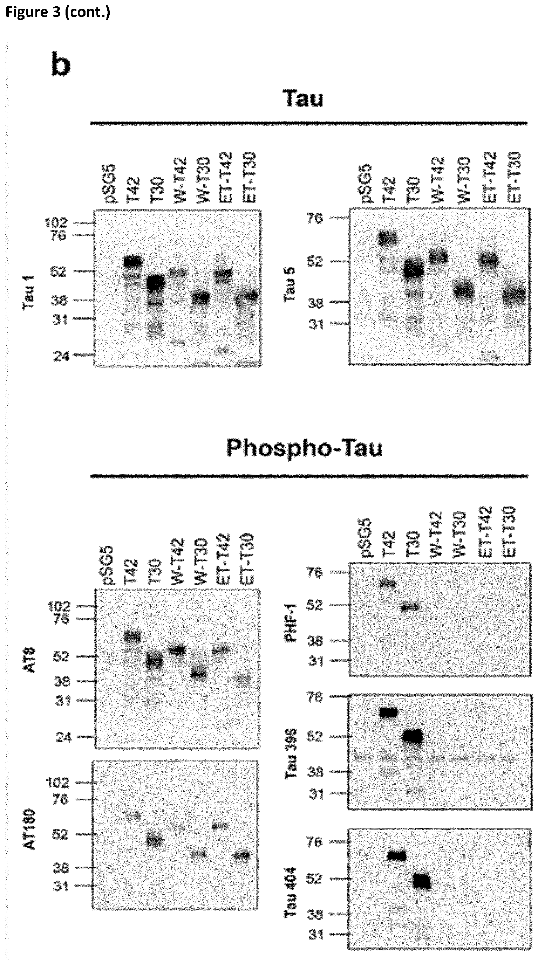

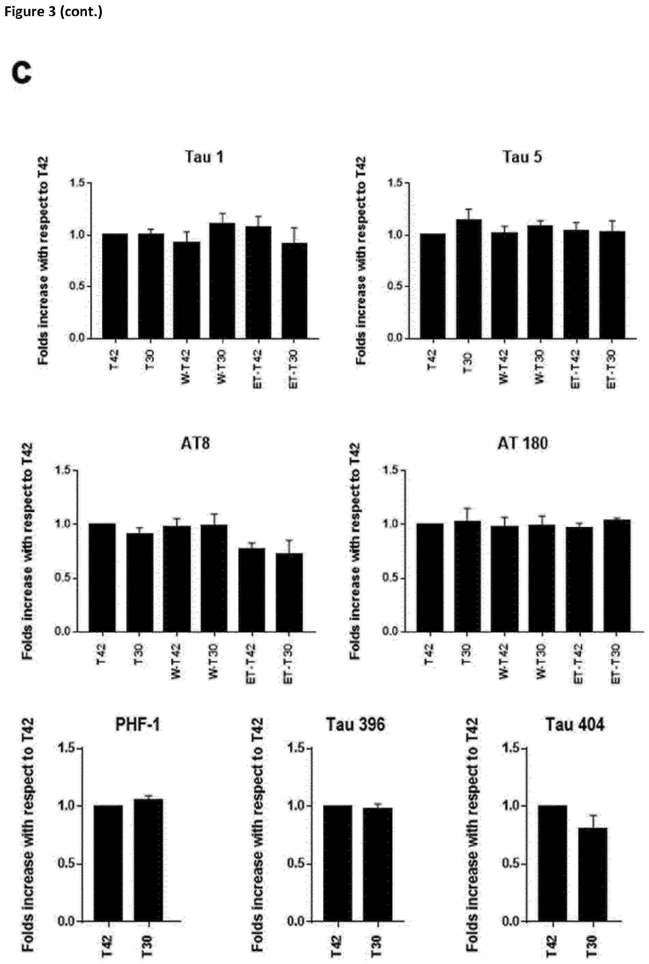

**Figure 3** **W-Tau phosphorylation pattern, a.** Schematic representation of different Tau protein isoforms: Full length isoform with 4 repeats (R) and two insertions (N) (T42), full length isoform with 3 repeats and no insertions (T30), the truncated by intron retention isoforms W-Tau with 4 repeats, two insertions and an extra peptide (W-T42) or with 3 repeats, no insertions and the extra peptide (W-T30); and the correspondent asparagine endopeptidase-truncated isoforms (ET-T42 and ET-T30). Representation of the antibodies recognizing the Tau molecule at their corresponding epitopes: Antibodies recognize all isoforms of Tau (Tau12 on amino acids 6-18; Tau 5 on amino acids 210-241 and Tau 7.51 on amino acids 315-376) or specific against dephosphorylated Tau in residues Ser195, 198, 199 and 202 (Tau1), and phospho-Tau in residues Ser202/Thr205 (AT8), Thr231 (AT180), Ser404 (Tau404), Ser396 (Tau396) and Ser396/Ser404 (PHF1). W-Tau antibody recognizes the unique peptide present on W-Tau isoforms. **b.** Samples from HEK293T cells transfected with the different isoforms were probed with different antibodies for phosphorylated and non-phosphorylated Tau. **c.** Quantification of the data of different Tau epitopes with respect to total Tau measured with Tau 7.51 antibody, showing mean and SEM (n=4). One-way ANOVA and Dunnett's test were performed to compare each isoform and T42 full-length level of phosphorylation.

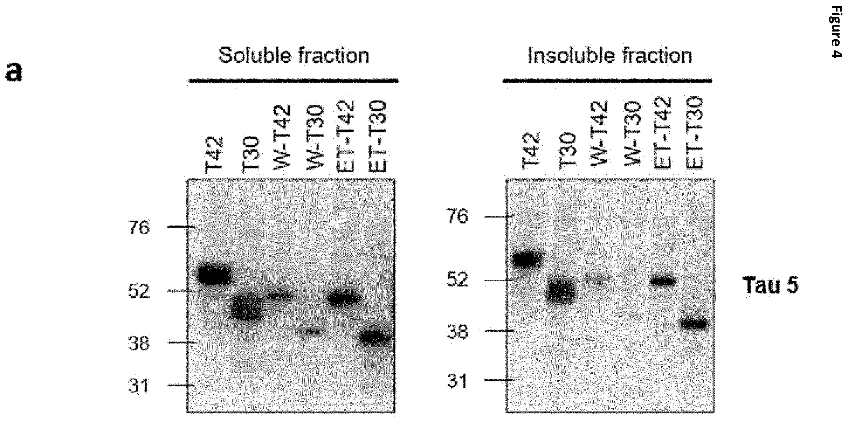

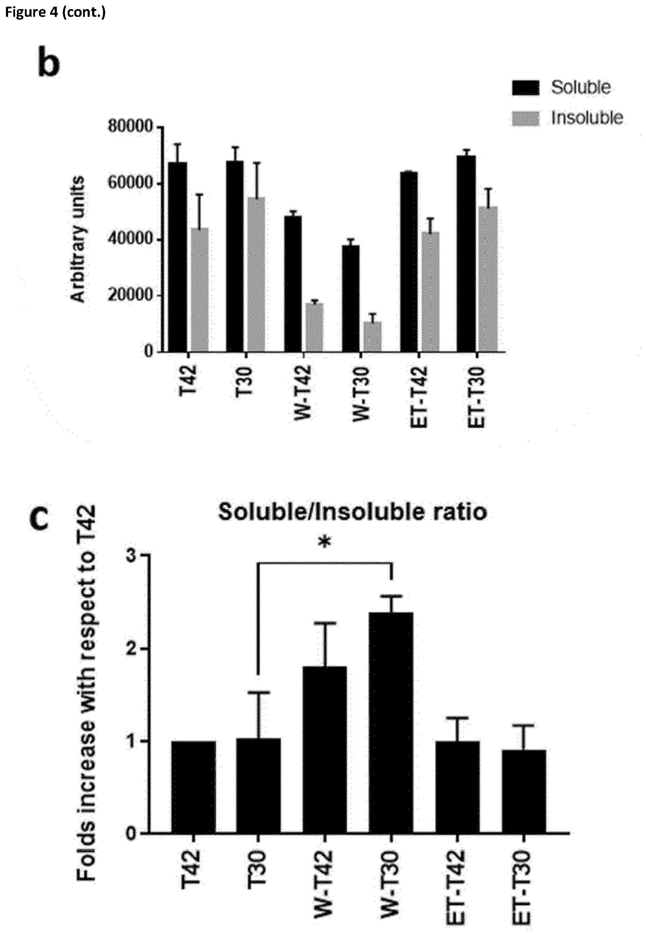

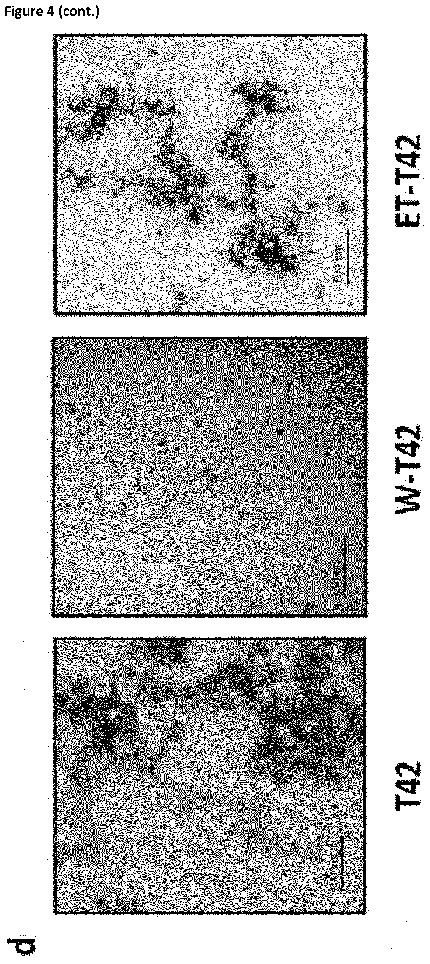

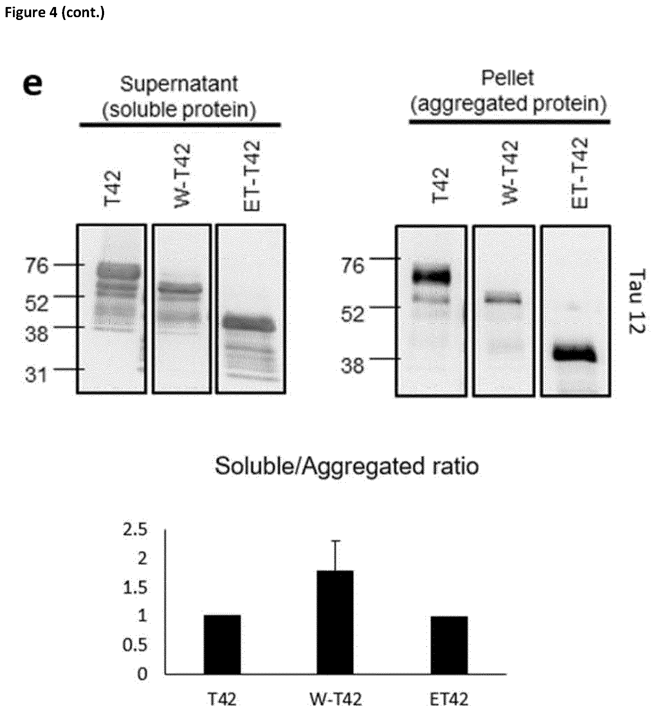

**Figure 4** **W-Tau aggregation capacity. a.** Representative Western blot of the presence of Tau in 1% sarkosyl-soluble and insoluble cell fractions of HEK293T cell overexpressing different Tau isoforms (T42, T30, W-T42, W-T30, ET-42 and ET-T30) detected with Tau 5 antibody **b-c.** Quantification of the signal obtained from Tau 5 showing sarkosyl-soluble vs insoluble fractions (b) or soluble/insoluble ratio (c). Graphs show means and SEM (N = 3). One-way ANOVA and Dunnett's multiple comparisons test were performed to compare each isoform with the correspondent full length isoform, * *p* ≤ 0.05. **d.** Representative electron microscopy images of Tau aggregates obtained upon *in vitro* incubation from partially purified T42, W-T42 and ET-42 extracts from HEK293T cells in the presence of heparin to prompt aggregates formation. Scale bars show 500 nm wide. **e.** Western blot showing soluble (supernatant) and aggregated (pellet) protein from the incubation in **d,** upon centrifugation to separate both fractions. Quantification shows the ratio between soluble and aggregated protein.

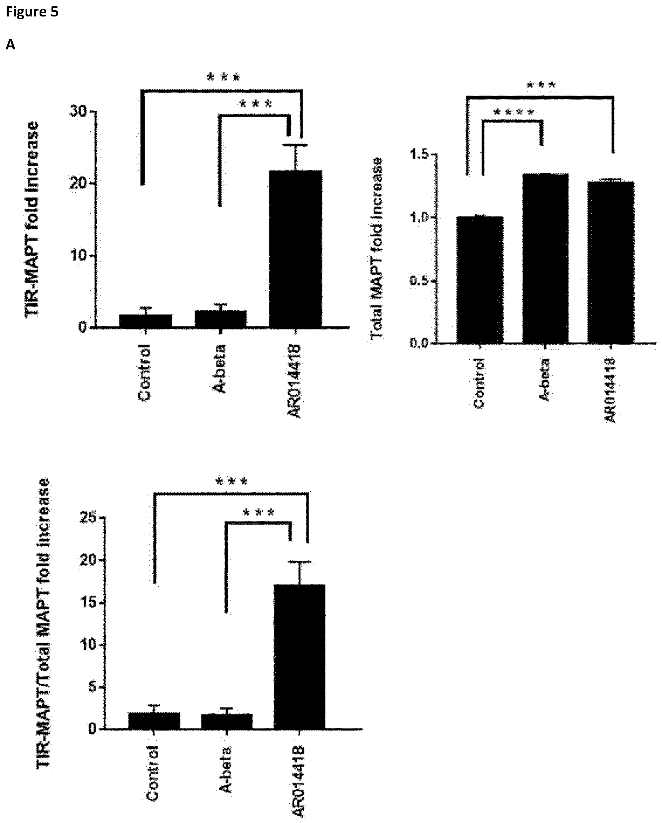

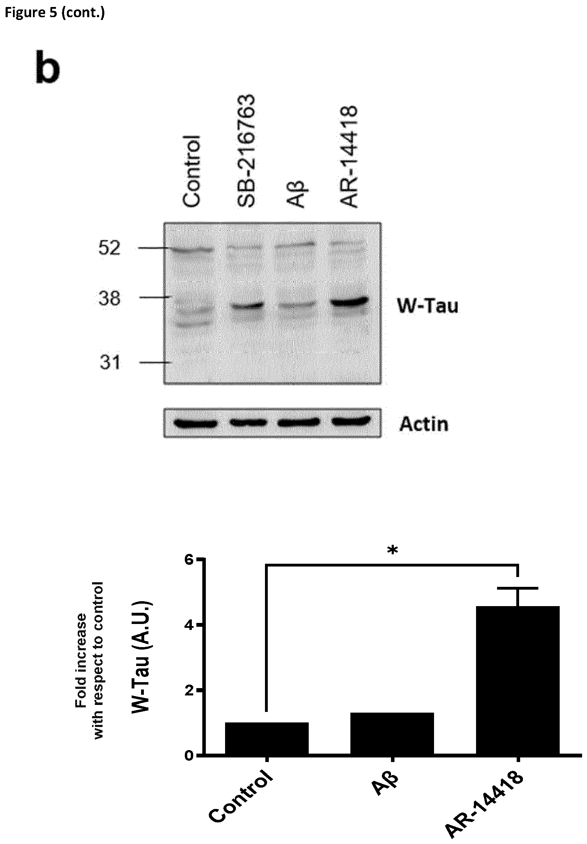

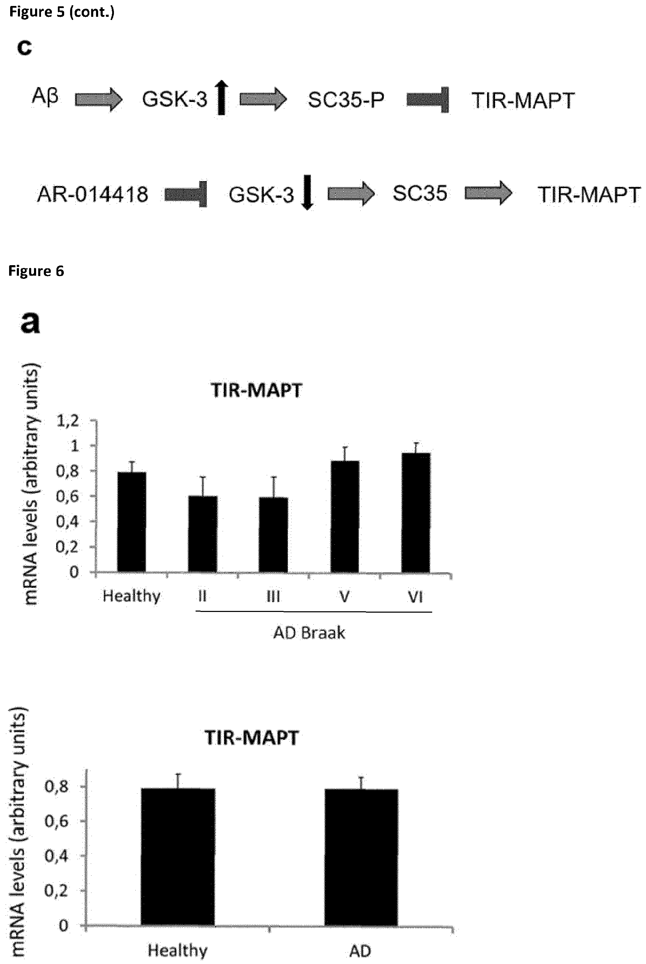

**Figure 5** **Modulation of *TIR-MAPT* and W-Tau by GSK3. a.** Detection of mature cytoplasmic RNA levels of TIR-MAPT, total MAPT and the ratio TIR-MAPT/total-MAPT in SK-N-MC human neuroblastoma cell line in the absence or presence of 5 µg/ml amyloid-β and the GSK-3 inhibitor AR-014418 (10 µM) for 24h. Graphs show mean and SEM. *** p ≤ 0.001; **** p ≤ 0.0001. **b.** Western blot analysis of the protein levels of W-Tau (W-Tau antibody) in SK-N-MC cells treated with SB216763 (25 µM), Aβ (5 µl) or AR-14418 (10 µM). Right panel shows the quantification of the signal of complete lanes obtained with W-Tau antibody. Graph show mean and SEM. One-way ANOVA for multiple comparisons followed by a Kruskal-Wallis test was performed to compare treated and untreated cells. *: *p* ≤ 0.05. **c.** Schematic representation of the modulation of *TIR-MAPT* by splicing factor SC35, regulated by GSK3-mediated phosphorylation. Blue arrows represent activation; red, truncated arrows represent inhibition

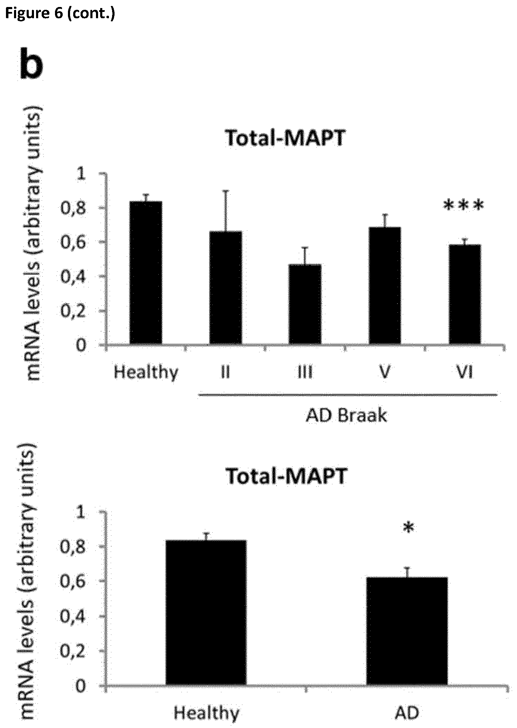

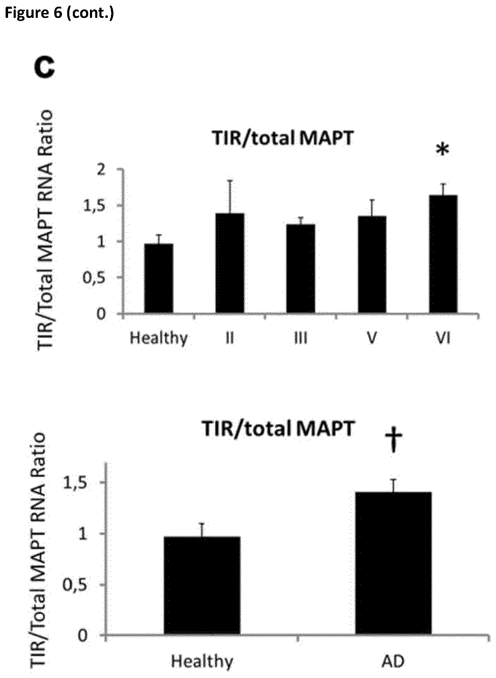

**Figure 6** ***TIR-MAPT* RNA expression in the brains of AD patients. a.** Measurement of *TIR-MAPT,* **b.** total-*MAPT* and **c.** *TIR-MAPT*/total*-MAPT* ratio of RNA levels by RT-qPCR of non-demented (n=8) and AD hippocampal samples (n=16) classified according to their Braak stage (Braak II n = 3, Braak III n = 2, Braak V n = 7 and Braak VI n = 4), and healthy vs. AD patients. Graphs show means and SE (*: p ≤ 0.05; *** p ≤ 0.001, †_{:} p = 0,078)

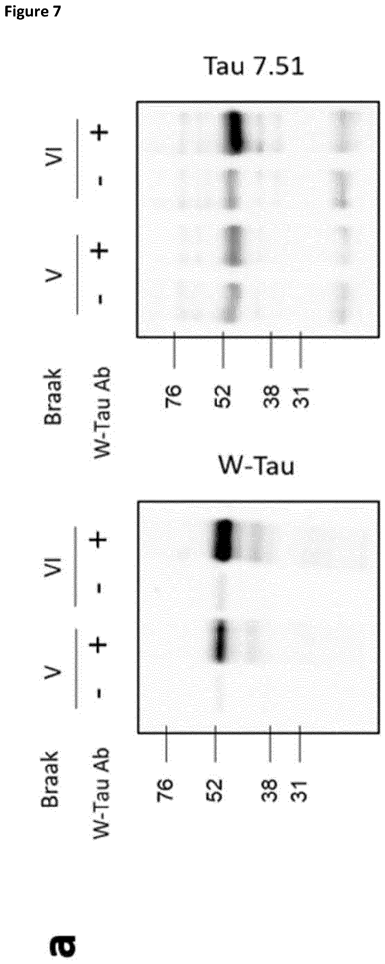

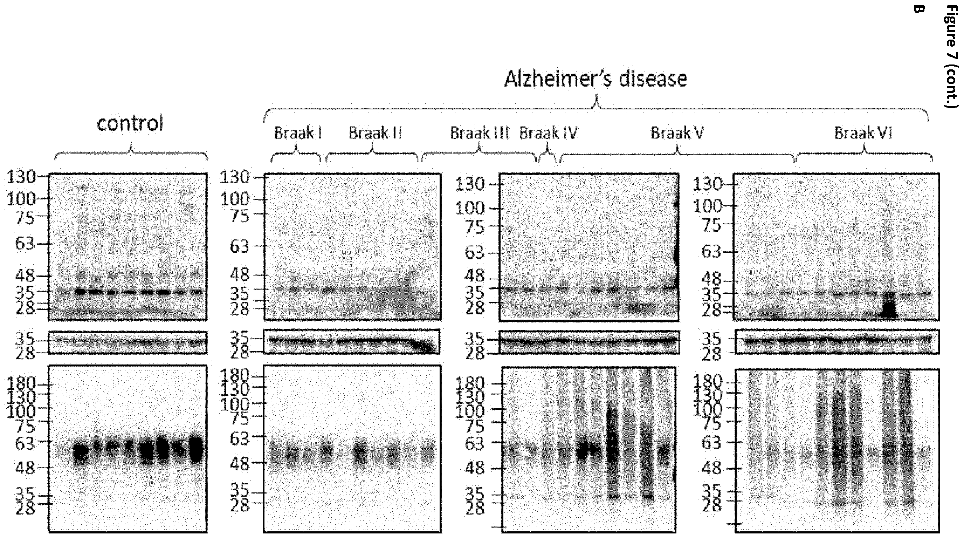

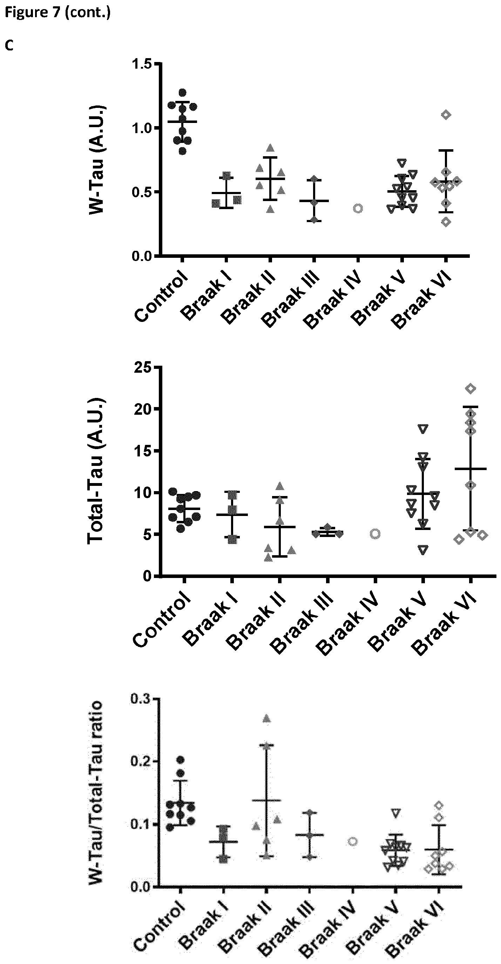

**Figure 7** **Tau protein determination in AD patients' brain. a.** Immunodetection of the presence of W-Tau isoforms using W-Tau antibody in two different frontal lateral cortex brain extracts derived from AD patients' brains (Braak V and VI, respectively). The immunoprecipitate was characterized by Westernblotting. Left panel shows the blot developed using W-Tau antibody; right panel using Tau 7.51 antibody. **b.** Western blot analysis of the levels of W-Tau and total Tau in frontal lateral cortical samples of non-demented (n=9) and AD patients classified according to their Braak stage (Braak I = 3; Braak II n=6, Braak III n=3, Braak IV n=1, and Braak V n=10, Braak VI n=8). c. Quantification of W-Tau and total Tau protein levels as well as W-Tau/total-Tau ratio of each group. One-way ANOVA and Dunnett's multiple comparisons test were performed and statistical significance of each group with respect to non-demented control individuals was given (* p ≤ 0.05; ** p ≤ 0.01; *** p ≤ 0.001; **** p ≤ 0.0001). *A. U.: Arbitrary Units*

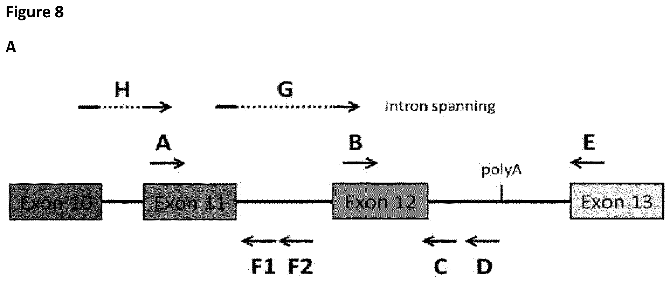

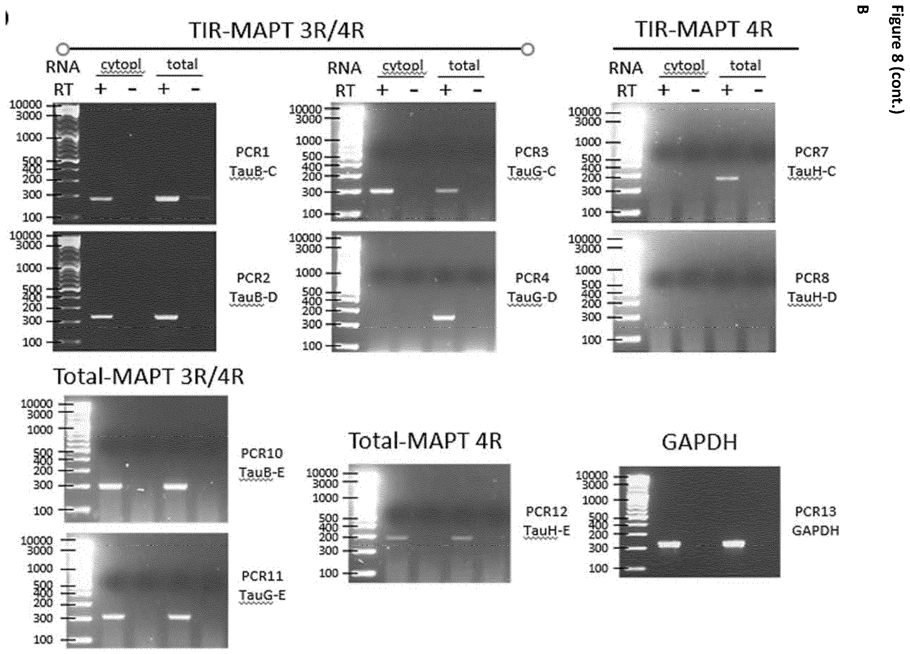

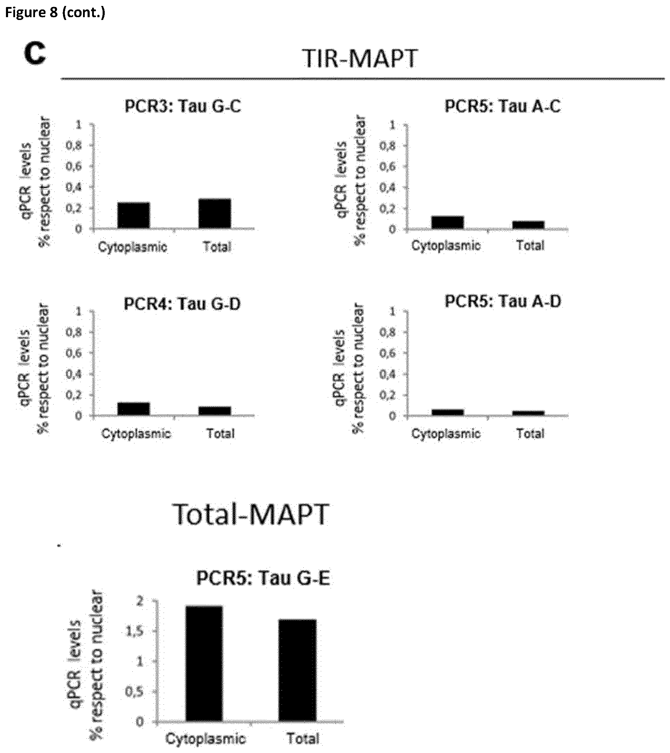

**Figure 8** **Semiquantitative and quantitative determination of W-Tau in SH- SY5Y.** a. Schematic representation of the hybridization sites of the primers (detailed in Supplementary Table 2) designed for semi-quantitative PCR in MAPT sequence. b. Representative images of agarose gels showing semi-quantitative PCR results using total or cytoplasmic-enriched mRNA of SH-SY5Y cells. Results showed the existence of RNA species from exon 12 to intron 12 (PCR 1 and 2) and from exon 11 to intron 12 where intron 11 was spliced out (PCR 3 and 4). On the contrary, RNA species from exon 10 to intron 12 where introns 10 and 11 were spliced out were not found in cytoplasmic mature enriched fraction (PCR 7 and 8). RNA species where intron 12 was spliced out were found in all fractions (PCR 10, 11 and 12). Controls of the addition (RT+) or no addition of retrotranscriptase (RT-) were included. PCR 13 amplifying GAPDH transcript was used as control. Detailed information of all semi-quantitative PCR combinations and amplicon sizes is provided in Supplementary Table 5. c. Quantitative RT-PCR analysis of the same analysis shown in b.

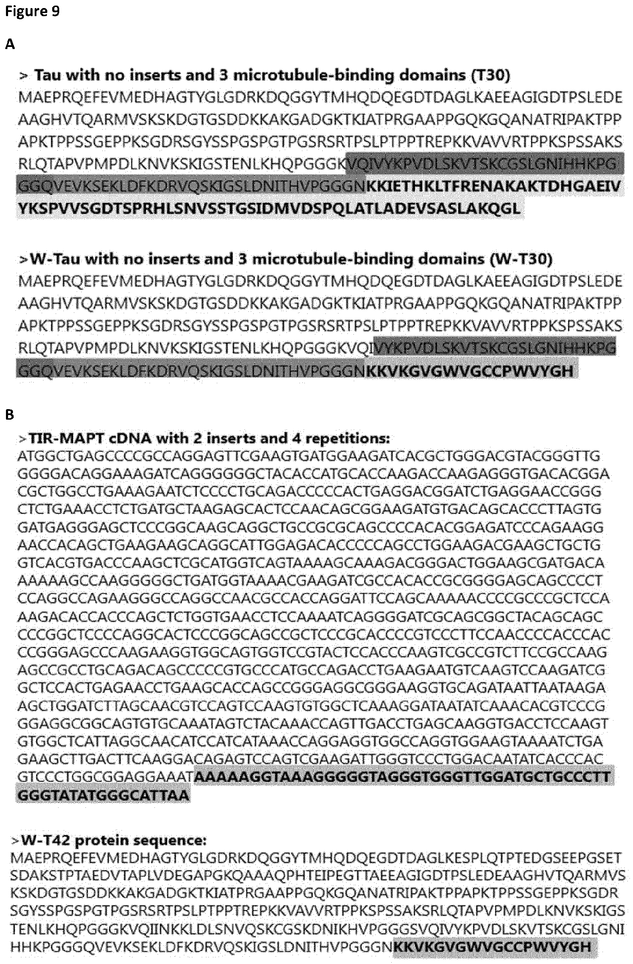

**Figure 9** **Comparison of predicted translation of MAPT canonical transcript and intron 12-retaining MAPT transcript.** a. Tau protein sequence for the isoform containing 3 tandem repeats without inserts, compared to the predicted translation of intron 12-retaining MAPT RNA sequences with 3 tandem repeats and without inserts. The highlighted fragments correspond to the exons and intron depicted in Fig. 1a. The difference between both sequences is marked in bold letters. b. Nucleotide cDNA sequence of TIR-MAPT and corresponding amino acid sequence of W-Tau with two inserts and 4 repeats (W-T42). The part of the sequence highlighted in orange indicates the TIR-MAPT or W-Tau specific sequence corresponding to intron 12 retention up to the stop codon TAA.

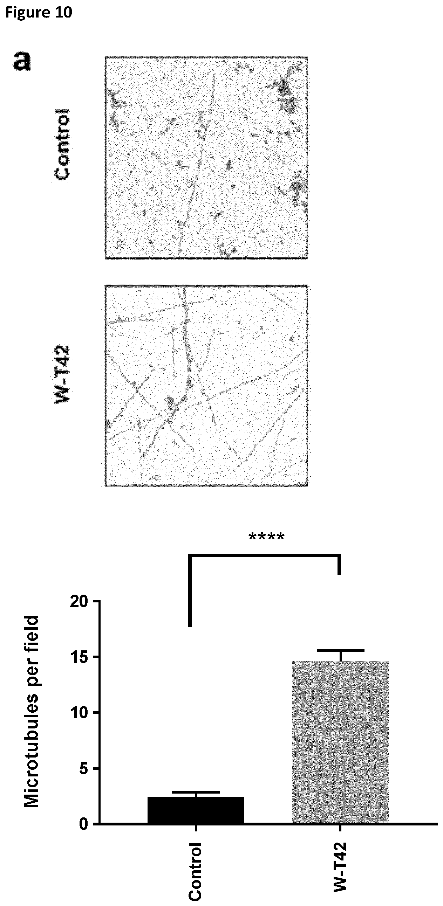

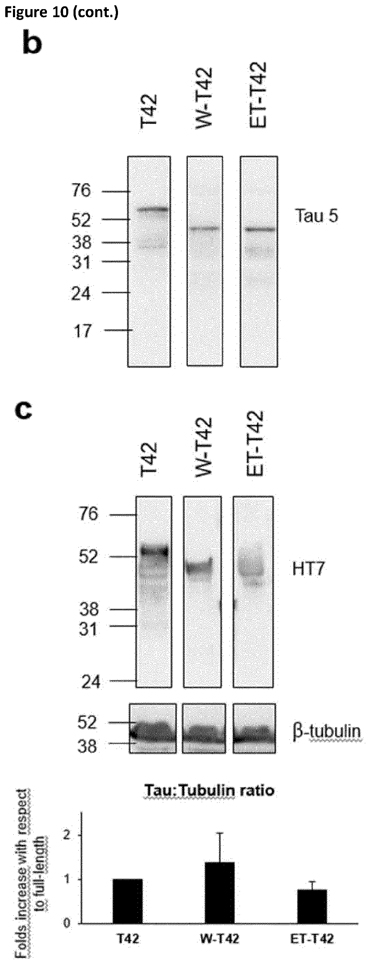

**Figure 10** **Microtubule binding capacity of W-Tau.** a. Electronic microscopy images comparing microtubule polymerization in the absence or presence of W-T42 isoform and corresponding quantification of microtubules per field. Seven fields were analyzed for control lacking W-T42 and 13 fields were analyzed for samples containing W-T42. Graph show mean and SEM (**** p ≤ 0.0001). b. Representative Western blot images of T42, W-T42 and ET-T42 purified from bacteria, as were used for microtubule-binding experiments. c. Western blot analysis of the microtubule-bound Tau after copolymerization of different human Tau isoforms with mouse brain microtubules detected with an anti-human Tau antibody (HT7). Quantification of Tau/tubulin ratio of each Tau isoform with respect to the full-length isoform (T42). Graph show means and SEM of three independent experiments for 4R isoforms.

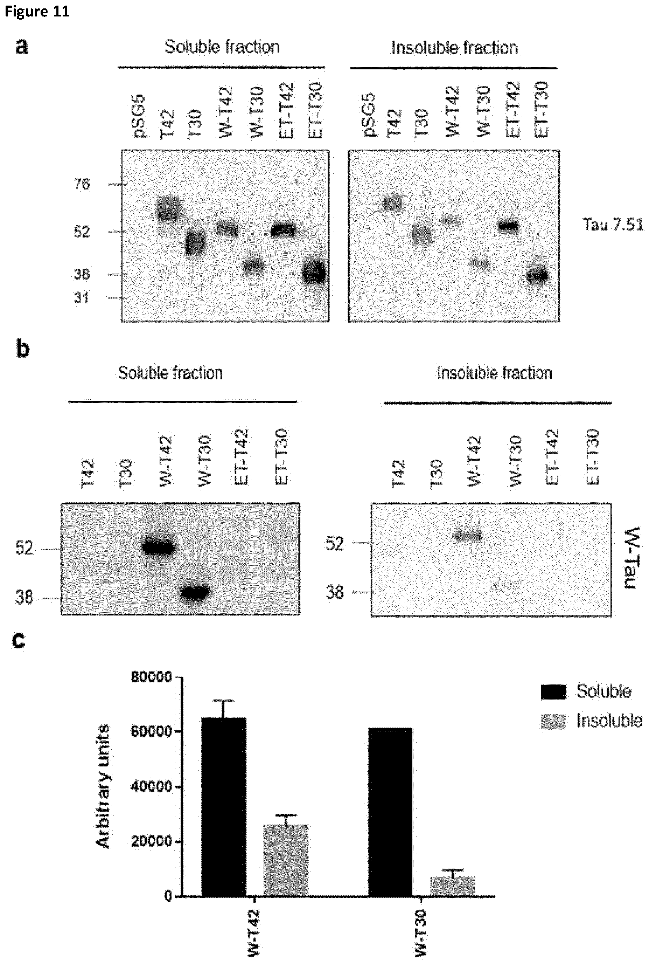

**Figure 11** **W-Tau aggregation capacity.** a. Representative Western blot of the presence of Tau in 1% Triton X-100-soluble and insoluble cell fractions of HEK293T cells overexpressing different Tau isoforms (T42, T30, W-T42, W-T30, ET-42 and ET-T30), detected with 7.51 antibody. b. Representative Western blot of the presence of W-Tau in 1% sarkosyl-soluble and insoluble cell fractions of HEK293T cell overexpressing different Tau isoforms (T42,T30, W-T42, W-T30, ET-42 and ET-T30) detected with W-Tau antibody. c. Quantification of the signal obtained with W-Tau antibody (N=2).

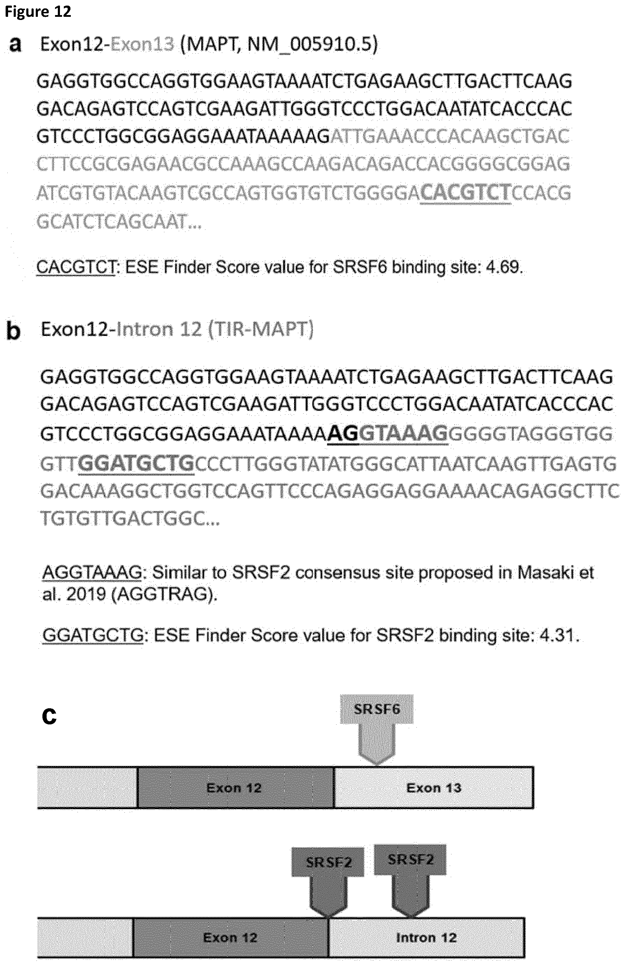

**Figure 12** **Study of potential splicing factors involved in the exclusion or inclusion of intron 12. Literature searching and in silico study using ESEFinder 3.0 (http://exon.cshl.edu/ESE/) of the exon 12-exon 13 or exon 12-intron 12 boundary of human MAPT** a. Sequence of exon 12-exon 13 boundary in MAPT RNA. Splicing factor binding site predicted by ESEFinder for SRSF6 is indicated. b. Sequence of exon 12-intron 12 human MAPT RNA, including the coding sequence expressing the extra peptide present in W-Tau. The exon 12-intron 12 junction is similar to the low-affinity SRSF2 binding sequence reported by Masaki et al, 2019 (AGGTAAAG), and ESEFinder predicts another SRSF2 binding site (GGATGCTG). c. Schematic representation of the splicing factors binding sites in full-length MAPT species versus TIR-MAPT species.

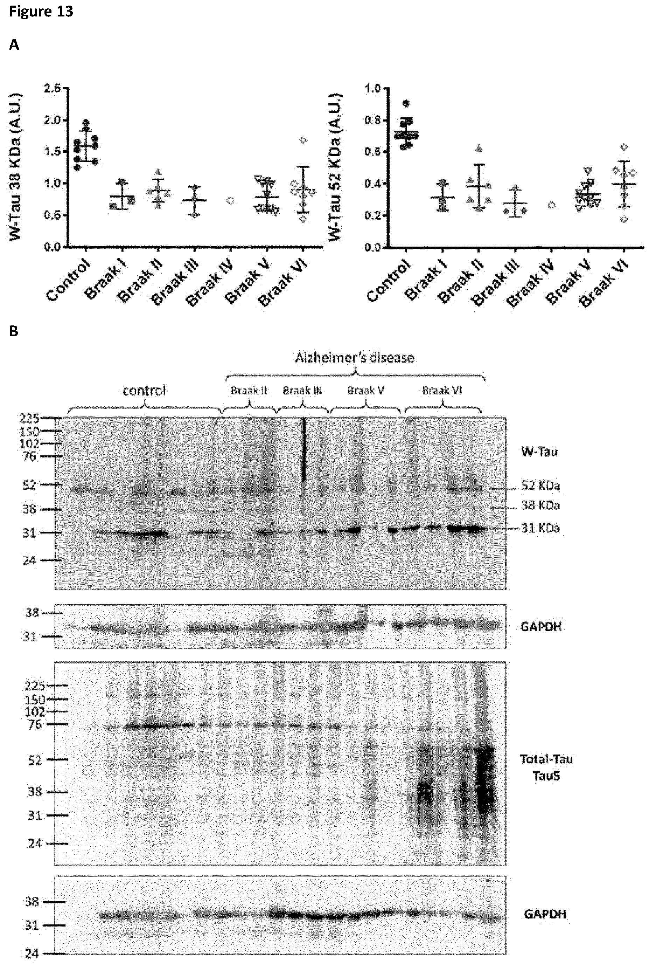

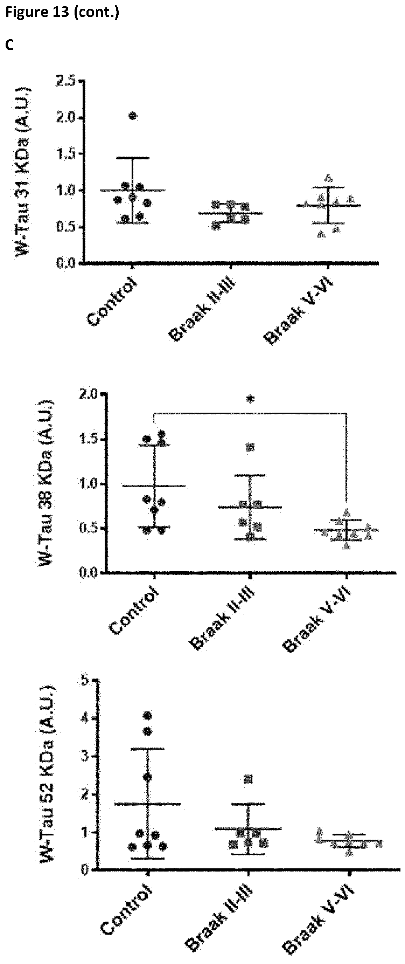

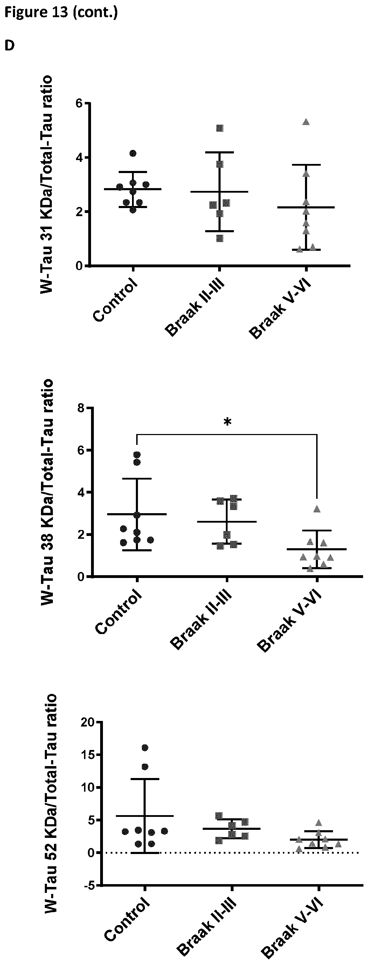

**Figure 13****. W-Tau expression in human brain** a. Quantification of W-Tau 38 KDa (W-T30) and 52 KDa band (W-T42) of the western blot shown in Fig. 7b of frontal lateral cortex samples of non-demented subjects (n=9) and AD patients classified according to their Braak stage (Braak I = 3; Braak II n=6, Braak III n=3, Braak IV n=1, and Braak V n=10, Braak VI n=8). b. Representative Western blot analysis of W-Tau and total-Tau (Tau5) expression in hippocampal brain samples of non-demented (n=9) and AD patients classified according to their Braak stage (Braak II n=3, Braak III n=3, Braak V n=4 and Braak VI n=4). c. Quantification of each W-Tau band 31 KDa (W-Tau truncated), 38 KDa (W-T30) or 52 KDa (W-T42) in non-demented (control) individuals, early-mild AD (Braak II-III) or severe AD (Braak V-VI). d. Similar quantification of the expression of W-Tau bands with respect to total-Tau expression. One-way ANOVA and Dunnett's multiple comparisons test were performed and statistical significance of each group with respect to non-demented control individuals was given (* p ≤ 0.05; ** p ≤ 0.01; *** p ≤ 0.001; **** p ≤ 0.0001). A.U.: Arbitrary Units.

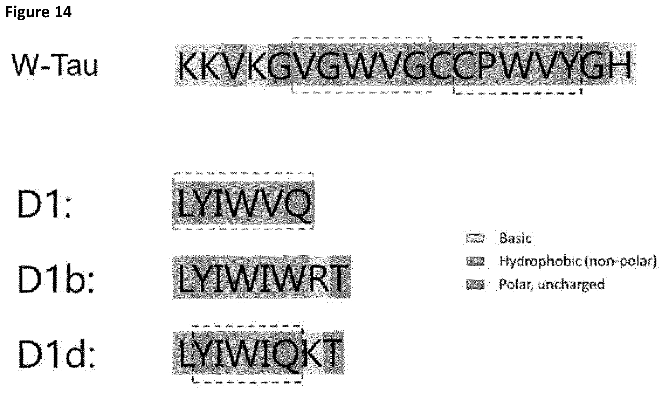

**Figure 14** Comparison of the amino acid sequence of the W-Tau peptide with those of different Tau-Aβ cross-seeding inhibitors. These inhibitors are described in Griner et al [26].

### Brief description of the invention

A first aspect of the invention refers to an isolated tau-isoform protein or peptide, or a fragment thereof, comprising at the C-terminus domain amino acid sequence WVGCCPW (SEQ ID NO 1) or amino acid sequence GVGWVG, wherein the C-terminus domain is understood as the 30, 25, 20 or preferably 18 amino acids sited at the end of the amino acid chain of the protein or peptide.

In a preferred embodiment of the first aspect of the invention, the said protein or peptide consists essentially of or consists of amino acid sequence WVGCCPW (SEQ ID NO 1) or of amino acid sequence GVGWVG.

In another preferred embodiment of the first aspect of the invention, the said protein or peptide comprises at the C-terminus domain amino acid sequence KKVKGVGWVGCCPWVYGH (SEQ ID NO 2), wherein preferably the C-terminus domain is understood as the 30, 25, 20 or preferably 18 amino acids sited at the end of the amino acid chain of the protein or peptide.

In another preferred embodiment of the first aspect of the invention, the said protein or peptide consists essentially of or consists of amino acid sequence KKVKGVGWVGCCPWVYGH (SEQ ID NO 2).

In another preferred embodiment of the first aspect of the invention, the said protein or peptide comprises, consists essentially of or consists of peptide sequence:

MAEPRQEFEVMEDHAGTYGLGDRKDQGGYTMHQDQEGDTDAGLKAEEAGIGDTPSLEDEAAGHVTQ
ARMVSKSKDGTGSDDKKAKGADGKTKIATPRGAAPPGQKGQANATRIPAKTPPAPKTPPSSGEPPKSGD
RSGYSSPGSPGTPGSRSRTPSLPTPPTREPKKVAVVRTPPKSPSSAKSRLQTAPVPMPDLKNVKSKIGSTEN
LKHQPGGGKVQIVYKPVDLSKVTSKCGSLGNIHHKPGGGQVEVKSEKLDFKDRVQSKIGSLDNITHVPGG
GNKKVKGVGWVGCCPWVYGH (SEQ ID NO 14).

In another preferred embodiment of the first aspect of the invention, the said protein or peptide comprises, consists essentially of or consists of peptide sequence:

MAEPRQEFEVMEDHAGTYGLGDRKDQGGYTMHQDQEGDTDAGLKESPLQTPTEDGSEEPGSETSDAK
STPTAEDVTAPLVDEGAPGKQAAAQPHTEIPEGTTAEEAGIGDTPSLEDEAAGHVTQARMVSKSKDGTG
SDDKKAKGADGKTKIATPRGAAPPGQKGQANATRIPAKTPPAPKTPPSSGEPPKSGDRSGYSSPGSPGTP
GSRSRTPSLPTPPTREPKKVAVVRTPPKSPSSAKSRLQTAPVPMPDLKNVKSKIGSTENLKHQPGGGKVQII
NKKLDLSNVQSKCGSKDNIKHVPGGGSVQIVYKPVDLSKVTSKCGSLGNIHHKPGGGQVEVKSEKLDFKD
RVQSKIGSLDNITHVPGGGNKKVKGVGWVGCCPWVYGH (SEQ ID NO 15).

A second aspect of the invention refers to a composition comprising one or a plurality of tau-isoform protein/s or peptide/s as defined in the first aspect of the invention or as defined in any of the preferred embodiments of the first aspect.

A third aspect of the invention refers to a composition comprising a polynucleotide encoding at least one tau-isoform protein or peptide as defined in the first aspect of the invention or as defined in any of the preferred embodiments of the first aspect. Preferably, the polynucleotide is disposed within a vector selected for its ability to express the protein or peptide in a mammalian cell, preferably in a human cell.

A fourth aspect of the invention refers to a composition as defined in any of the second or third aspects of the invention, for use in a method of inhibiting the formation of Ab aggregates or tau aggregates in a subject in need thereof.

A fifth aspect of the invention refers to a composition as defined in any of the second or third aspects of the invention, for use in a method of inhibiting the development or progression of Ab plaque formation in a subject in need thereof.

A sixth aspect of the invention refers to a composition as defined in any of the second or third aspects of the invention, for use in a method of inhibiting the development or progression of a tauopathy in a subject in need thereof.

A seventh aspect of the invention refers to a composition as defined in any of the second or third aspects of the invention, for use in a method of inhibiting the development or progression of Alzheimer's disease in a subject in need thereof.

An eighth aspect of the invention refers to a composition comprising an effective amount of the protein as defined in the second aspect or the polynucleotide as defined in the third aspect, for use in a method of treatment of a subject having an amyloid disease characterized by aggregation of Tau protein.

A ninth aspect refers to a pharmaceutical composition comprising a composition as defined in the second or third aspect of the invention, and a pharmaceutically acceptable carrier including a peptide stabilizing excipient. Preferably, the peptide stabilizing excipient comprises a preservative, a tonicity adjusting agent, a detergent, a hydrogel, a viscosity adjusting agent, or a pH adjusting agent.

A tenth aspect refers to an *in vitro* use of the isolated tau-isoform protein or peptide as defined in the first aspect of the invention or as defined in any of the preferred embodiments of the first aspect, as a biomarker for the prognosis of an amyloid disease characterized by aggregation of Tau protein in a human subject.

An eleventh aspect of the invention refers to an in vitro use of the isolated tau-isoform protein or peptide as defined in the first aspect of the invention or as defined in any of the preferred embodiments of the first aspect, as a biomarker for the prognosis of Alzheimer's disease in a human subject.

### Detailed description of the invention

In the description of embodiments, reference may be made to the accompanying figures which form a part hereof, and in which is shown by way of illustration a specific embodiment in which the invention may be practiced. It is to be understood that other embodiments may be utilized, and structural changes may be made without departing from the scope of the present invention. Many of the techniques and procedures described or referenced herein are well understood and commonly employed by those skilled in the art. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

Although recent evidence point out that a large proportion of predicted alternative transcripts might not be translated into proteins [55], *MAPT* gene has been proven to be genuinely subjected to alternative splicing [1, 55]. Also, *MAPT* contains a cryptic exonic sequence codifying for the protein saitohin nested in the Tau gene within intron 9 [14]. Thus, the removal of RNA sequences in Tau RNA is very usual, but the translation of intronic sequences could also take place. Curiously, it has been suggested that the region of "big" Tau corresponding to exon 4a arose, evolutionary, from an intron from another protein [20].

Tau alternative splicing is also regulated by several features such as development, aging, disease or brain region [8, 54, 56, 58]. Tau isoforms lacking exons 2, 3 and 10, can be mainly found at early developmental stages [2, 56] and alternative splicing of exon 10 has been related to some tauopathies [10, 50].

Recently, a toxic fragment containing residues 1-368 has been reported [63]. Since residue 368 coincides with the end of exon 12, in this invention we have studied whether a failure in the removal of intron 12 could give rise to a novel Tau isoform. We found that intron 12 retention can take place, in low proportions in non-demented human control as well as AD patients' brains. The failure of intron 12 removal results in the appearance of a Tau isoform containing 18 additional residues after exon 12. Strikingly, this new isoform is significantly reduced in AD, especially in the advanced stages, while total Tau is accumulated. It is worth emphasizing that this isoform is human-specific, since other species' Tau orthologues do not contain the polyadenylation canonical site that allows intron retention to give rise to a truncated protein. This is especially noteworthy considering that Alzheimer's disease is a human pathology that is not well reproduced in animal models.

A possible mechanism explaining intron 12 retention is that trans-acting splicing factors could be modified, for example by phosphorylation, and that the modified factor could change its function. Moreover, modification of intron splicing has been previously reported for SR-proteins [40] and, specifically, for SRSF2 (SC35) which, when phosphorylated by GSK3 kinase, favors the expression of Tau 3R (lacking exon 10) [29]. Additionally, it has been described that the absence of SRSF2 favors exon 10 inclusion increasing Tau 4R species [12]. Notably, intron splicing events have been previously reported for SRSF2 and other member of the SR-proteins family [40]. In this invention, we found that GSK3 inhibition also induces the retention of intron 12, presumably by the inhibition of SRSF2 function, inducing the expression of *TIR-MAPT* mRNA and protein, while total Tau levels remain unmodified. SRSF2 could play a role in this process, since there are two open recognition sites for this factor in *TIR-MAPT* sequence*,* one of them located at the exon 12 - intron 12 junction [11, 38]. Of note, W-Tau levels did not change in amyloid-treated cultured cells, even though amyloid beta peptide has been reported to stimulate GSK3 [12, 28]. This may be due to GSK3 being constitutively active and, consequently, amyloid-mediated activation does not imply further SRSF2 phosphorylation when comparison to untreated cells.

The novel Tau fragment described in this invention is somehow similar to the previously reported fragment generated by cleavage of AEP [63]. However, contrary to what was expected, the isoforms truncated by intron retention exhibit a potentially protective behavior, with decreased aggregation capacity but conservation of microtubule assembly capacity. This novel Tau isoforms contain a unique 18-aminoacid extra peptide, including the sequence GVGWVG, **(****Figure 14****).** Thus, we suggest that the decreased aggregation capacity of W-Tau could be related to the presence of this extra peptide. Moreover, W-Tau lacks the 12 amino acids after the fourth repeat, present in exon 13, which are found in the core of Tau filaments isolated from the brain of patients suffering some tauopathies, including Alzheimer's disease [62]. It is also worth noting that the specific W-Tau 18 amino acid sequence contains two adjacent cysteines (KKVKGVGWVGCCPWVYGH), whose oxidation to cystine can mediate the formation of a strained eight-member ring [47], a conformation that would substantially differ of that of other Tau isoforms and that has been proposed to be able to affect protein function and conformation stability [33, 39].

As for the relevance of this novel truncated isoform in AD's development and progression, similar *TIR-MAPT* mRNA levels were found in non-demented control subjects and AD patients' samples. However, on a protein level, we have detected higher levels of W-Tau protein in healthy individuals compared to AD patients. Discordant RNA and protein levels for Tau in AD have been previously described [23]. We hypothesize that, in this case, it may be related to a relatively faster turnover for W-Tau isoforms, maybe linked to a different conformation rising from its different sequence. These data also showed a clearly less aggregative pattern on W-Tau isoforms when compared to total Tau **(****Fig. 7****),** suggesting that this is a less aggregation-prone Tau isoform indeed.

Finally, therapeutic correction of aberrant splicing that could take place in Alzheimer's disease has been suggested for some nuclear RNAs [4, 31]. Given the potential role of this new Tau truncated isoform on a possible AD therapy, we will look for future therapeutic strategies based on an increase of this less aggregation-prone Tau isoform or the prevention of its decrease.

Therefore, herein we report peptide-based inhibitors that reduce or prevent amyloid beta (Ab) and/or tau aggregation and toxicity, preferably of already-aggregated species, and methods of using these peptide-based inhibitors. It is noted, that this invention is especially suitable for treating diseases such as Alzheimer's disease (AD) characterized by plaques of amyloid beta (Ab) and neurofibrillary tangles of tau. Ab aggregation is thought to occur at early stages of the disease, and ultimately gives way to the formation of tau tangles which track with cognitive decline.

Embodiments of the invention include, for example, a composition of matter comprising at least one peptide inhibitor (from hereinafter "peptide inhibitor/s of the invention") that reduces or prevents amyloid beta (Ab) and/or tau aggregation and toxicity, preferably of already-aggregated species, wherein the at least one peptide inhibitor comprises or consists of an amino acid sequence WVGCCPW (SEQ ID NO 1), KKVKGVGWVGCCPWVYGH (SEQ ID NO 2), GVGWVG (SEQ ID NO 3), GWVGCCPW (SEQ ID NO 4), VGWVGCCPW (SEQ ID NO 5), GVGWVGCCPW (SEQ ID NO 6), KGVGWVGCCPW (SEQ ID NO 7), VKGVGWVGCCPW (SEQ ID NO 8), WVGCCPWV (SEQ ID NO 9), GWVGCCPWV (SEQ ID NO 10), WVGCCPWVY (SEQ ID NO 11), WVGCCPWVYG (SEQ ID NO 12) or WVGCCPWVYGH (SEQ ID NO: 13) or any combination thereof; Preferably, wherein the at least one peptide inhibitor comprises or consists of amino acid sequence WVGCCPW (SEQ ID NO 1), KKVKGVGWVGCCPWVYGH (SEQ ID NO 2), or GVGWVG (SEQ ID NO 3); more preferably, wherein the at least one peptide inhibitor comprises or consists of amino acid sequence KKVKGVGWVGCCPWVYGH (SEQ ID NO 2). Preferably, the peptide inhibitor/s of the invention consists of or are comprised in a tau-isoform protein or peptide, or in a fragment thereof, wherein preferably the peptide inhibitor/s of the invention comprised or consists of peptide sequence:
MAEPRQEFEVMEDHAGTYGLGDRKDQGGYTMHQDQEGDTDAGLKAEEAGIGDTPSLEDEAAGHVTQ ARMVSKSKDGTGSDDKKAKGADGKTKIATPRGAAPPGQKGQANATRIPAKTPPAPKTPPSSGEPPKSGD RSGYSSPGSPGTPGSRSRTPSLPTPPTREPKKVAVVRTPPKSPSSAKSRLQTAPVPMPDLKNVKSKIGSTEN LKHQPGGGKVQIVYKPVDLSKVTSKCGSLGNIHHKPGGGQVEVKSEKLDFKDRVQSKIGSLDNITHVPGG GNKKVKGVGWVGCCPWVYGH (SEQ ID NO 14). Preferably,
Preferably, the peptide inhibitor/s of the invention comprised or consist of peptide sequence:

MAEPRQEFEVMEDHAGTYGLGDRKDQGGYTMHQDQEGDTDAGLKESPLQTPTEDGSEEPGSETSDAK
STPTAEDVTAPLVDEGAPGKQAAAQPHTEIPEGTTAEEAGIGDTPSLEDEAAGHVTQARMVSKSKDGTG
SDDKKAKGADGKTKIATPRGAAPPGQKGQANATRIPAKTPPAPKTPPSSGEPPKSGDRSGYSSPGSPGTP
GSRSRTPSLPTPPTREPKKVAVVRTPPKSPSSAKSRLQTAPVPMPDLKNVKSKIGSTENLKHQPGGGKVQII
NKKLDLSNVQSKCGSKDNIKHVPGGGSVQIVYKPVDLSKVTSKCGSLGNIHHKPGGGQVEVKSEKLDFKD
RVQSKIGSLDNITHVPGGGNKKVKGVGWVGCCPWVYGH (SEQ ID NO 15).

Optionally the composition comprises a plurality of peptide inhibitor/s of the invention. In certain embodiments of the invention, the peptide inhibitor sequence is coupled to a plurality of heterologous amino acids, for example a linker amino acid sequence and/or a cell penetrating peptide (CPP) amino acid sequence, this specific embodiment shall be explained in more detail below.

In certain embodiments of the invention, it is noted that the peptide inhibitor/s of the invention consists of or is comprised in a tau-isoform protein or peptide, or in a fragment thereof, and the peptide inhibitor of the invention is preferably located at the C-terminus domain or at the C-terminus motif or sequence (also known as the carboxyl-terminus, carboxy-terminus, C-terminal tail, C-terminal end, or COOH-terminus) of the tau-isoform protein or peptide, or of the fragment thereof. C-terminus is understood as the end of an amino acid chain (protein or polypeptide), terminated by a free carboxyl group (-COOH); therefore, preferably the peptide inhibitor/s of the invention is comprised within the last 30 amino acids of the amino acid chain, preferably within the last 25 amino acids of the amino acid chain, preferably within the last 20 amino acids of the amino acid chain, more preferably within the last 18 amino acids of the amino acid chain.

Another embodiment of the invention is a composition of matter comprising a polynucleotide encoding at least one of the peptide inhibitor/s of the invention. Typically, these polynucleotides are disposed within a vector selected for its ability to express the peptide inhibitor in a mammalian cell. In certain embodiments of the invention, the composition comprises a vector disposed within a mammalian cell.

In certain embodiments of the invention, the peptide inhibitor/s of the invention or a protein or peptide comprising the same, is typically less than 400 amino acids in length, less than 300 amino acids in length, less than 250 amino acids in length, less than 200 amino acids in length, less than 150 amino acids in length, less than 100 amino acids in length, less than 50 amino acids in length, less than 25 amino acids in length, less than 20 amino acids in length, more preferably less or equal to 18 amino acids in length.

As noted above, in certain embodiments of the invention, the peptide inhibitor/s of the invention or a protein or peptide containing the same may be coupled to heterologous amino acids such as a cell penetrating peptide (CPP) amino acid sequence, typically one less than 30 amino acids in length. Optionally the peptide is coupled to the heterologous amino acids by a peptide linker comprising 1-7 amino acids. In some embodiments of the invention, the heterologous amino acids forms a polycationic structure. In other embodiments of the invention, the heterologous amino acids forms an amphipathic structure. Embodiments of the invention include peptides wherein the peptide comprises at least one D amino acid (e.g. a peptide comprising all D-amino acids). Embodiments of the invention can further compare metabolic stability and efficacy of L- and D form peptide inhibitors.

Embodiments of the invention include coupling of at least one of the peptide inhibitor/s of the invention by chemical or biological (genetic) means to proteins or peptides to carry the peptide inhibitor across the blood-brain-barrier (BBB), as a therapy for neurodegeneration or movement disorder (it is noted that such "coupled" peptides shall also be understood as forming part of the definition of "peptide inhibitor/s of the invention"). For example, embodiments of the invention include coupling of any of the peptide inhibitor/s of the invention to a cell penetrating peptide and then coupling the resulting peptide by chemical or biological (genetic) means to proteins or peptides to carry the inhibitor across the blood-brain-barrier (BBB), as a therapy for neurodegeneration or movement disorder. Embodiments of the invention also include coupling of any of the peptide inhibitor/s of the invention to small molecules that aid in the ability of the peptide inhibitors to cross the BBB and/or cell membranes. Embodiments of the invention also include coupling of any of the peptide inhibitor/s of the invention to enzymes or small molecules (e.g. fluorophores) to aid in the diagnosis of pathology (ante and/or postmortum). Embodiments of the invention also include coupling of any of the peptide inhibitor/s of the invention to small molecules that aid in the ability of the peptide inhibitors to degrade amyloid aggregates.

Embodiments of the invention also include coupling of any of the peptide inhibitor/s of the invention by chemical means to a nano-particle capable of crossing the BBB and possibly also entering into cells, for example for use as a therapy for neurodegenation or movement disorder. Embodiments of the invention also include coupling of any of the peptide inhibitor/s of the invention by chemical means to a metal-containing nano-particle capable of crossing the BBB to create a diagnostic/biomarker for MRI or PET diagnosis of neurodegenerative disease or movement disorders (e.g. Parkinson's disease).

Embodiments of the invention also include insertion of any of the peptide inhibitor/s of the invention into the CDRs of an antibody to produce inhibitors of greater potency and/or specificity. The antibody can be a full antibody, and Fab domain, or a single-chain antibody. Coupling of the resulting antibody to a nano-particle provides embodiments of the invention useful for therapy or diagnosis as noted above.

A peptide inhibitor/s of the invention can be synthesized (e.g., chemically or by recombinant expression in a suitable host cell) by any of a variety of art-recognized methods. In order to generate sufficient quantities of an inhibitory peptide for use in a method of the invention, a practitioner can, for example, using conventional techniques, generate nucleic acid (e.g., DNA) encoding the peptide and insert it into an expression vector, in which the sequence is under the control of an expression control sequence such as a promoter or an enhancer, which can then direct the synthesis of the peptide. For example, one can (a) synthesize the DNA de novo, with suitable linkers at the ends to clone it into the vector; (b) clone the entire DNA sequence into the vector; or (c) starting with overlapping oligonucleotides, join them by conventional PCR-based gene synthesis methods and insert the resulting DNA into the vector. Suitable expression vectors (e.g., plasmid vectors, viral, including phage, vectors, artificial vectors, yeast vectors, eukaryotic vectors, etc.) will be evident to skilled workers, as will methods for making the vectors, inserting sequences of interest, expressing the proteins encoded by the nucleic acid, and isolating or purifying the expressed proteins. In illustrative embodiments of the invention, capping inhibitor peptides are loaded into adeno-associated virus (AAV) capsids and hydrogel-based polymers to mediate delivery across the blood brain barrier

One aspect of the invention is a method for reducing or inhibiting Tau aggregation, comprising contacting Tau amyloid protofilaments with an effective amount of at least one of the peptide inhibitor/s of the invention. Such a method can be carried out in vitro (in solution) or in vivo (e.g. cells in culture or in a subject). Another aspect of the invention is a method for reducing or inhibiting Ab aggregation, comprising contacting Ab proteins with an effective amount of one or more of the peptide inhibitor/s of the invention. Such a method can be carried out in vitro (in solution) or in vivo (e.g. cells in culture or in a subject).

Another aspect of the invention is a method for reactivating or restoring a biological or biochemical activity (function) of Tau and/or Ab which results from aberrant conformation of the Tau and/or Ab proteins. The method comprises contacting the Tau and/or Ab protein molecule having an aberrant conformation with an effective amount of the peptide inhibitor/s of the invention. As a result of contacting the Tau and/or Ab protein having the aberrant conformation, the lost biological or biochemical activity of the Tau and/or Ab molecule is reactivated or restored.

Another aspect of the invention is a method for inhibiting or preventing a loss of a biological or biochemical activity (function), of a Tau and/or Ab protein which results from aberrant conformation of the Tau and/or Ab protein. The method comprises contacting the Tau and/or Ab protein molecule having an aberrant conformation with an effective amount of the peptide inhibitor/s of the invention. As a result of contacting the Tau and/or Ab protein having the aberrant conformation, the loss of activity of the Tau and/or Ab molecule is inhibited or prevented.

Another aspect of the invention is a method for treating a subject having a disease or condition which is mediated by loss of function of Tau and/or Ab, such as a pathological syndrome in which Tau and/or Ab has an abnormal conformation (e.g. is aggregated or misfolded). That is, the pathological syndrome is associated with Tau and/or Ab having an aberrant conformation. The method comprises administering to the subject an effective amount of one or more peptides of the invention. In some embodiments, a cocktail of two of more of the peptide inhibitor/s of the invention is used.

Yet another embodiment of the invention is a method of observing the presence or absence of Tau amyloid fibrils and/or Ab in a biological sample comprising combining a biological sample with the peptide inhibitor/s of the invention that binds to Tau or Ab, allowing the peptide to bind to Tau amyloid fibrils and/or Ab that may be present in the biological sample, and then monitoring this combination for the presence of complexes formed between Tau amyloid fibrils and/or Ab and the peptide; wherein the presence of said complexes show the presence of Tau amyloid fibrils and/or Ab in the biological sample. Optionally in this method, the presence of complexes formed between Tau amyloid fibrils and/or Ab and the peptide is monitored using a detectable label that is coupled to the peptide (e.g. a heterologous peptide tag). Typically, the method is performed on a biological sample obtained from an individual suspected of suffering from a tauopathy. Such embodiments of the invention can be used, for example, in diagnostic methods designed to observe the presence or status of Alzheimer's disease, for example to detect disease beginnings before clinical symptoms, and to follow the effectiveness (or lack of effectiveness), of a therapeutic treatment.

Active variants of the inhibitory peptides described above are also included in the present invention. An "active variant" is a variant which retains at least one of the properties of the peptide inhibitor/s of the invention (e.g., the ability to inhibit, reduce or prevent Ab or Tau aggregation (i.e. fibrillation and/or tau cytotoxicity, wherein aggregation is understood as the formation of aggregates including fibers or fibrils)). Fibrilization, as used herein, refers to the formation of fiber or fibrils, such as amyloid fibrils.

Suitable active variants include peptidomimetic compounds (any compound containing non-peptidic structural elements that is capable of mimicking the biochemical and/or biological action(s) of a natural mimicked peptide), including, for example, those designed to mimic the structure and/or binding activity (such as, for example, hydrogen bonds and hydrophobic packing interactions) of the peptides according to the methods disclosed herein).

In one embodiment, active variants of the peptide inhibitor/s of the invention are shortened by 1-3 (e.g., 1, 2 or 3) amino acids at either the N-terminus, the C-terminus, or both of the starting inhibitory peptide. In another embodiment, the active variants are lengthened (extended) by 1, 2, 3 or 4 amino acids at the C-terminal end of the starting inhibitory peptide. A variety of other types of active variants are included in embodiments of the invention. In some embodiments, amino acids other than the ones noted above are substituted. These amino acids can help protect the peptide inhibitors against proteolysis or otherwise stabilize the peptides, and/or contribute to desirable pharmacodynamic properties in other ways. In some embodiments, the non-natural amino acids allow an inhibitor to bind more tightly to the target because the side chains optimize hydrogen bonding and/or apolar interactions with it. In addition, non- natural amino acids offer the opportunity of introducing detectable markers, such as strongly fluorescent markers which can be used, e.g., to measure values such as inhibition constants. Also included are peptide mimetics, such as, e.g., peptoids, beta amino acids, N-ethylated amino acids, and small molecule mimetics.

In one embodiment, non-natural amino acids are substituted for amino acids in the sequence. More than 100 non-natural amino acids are commercially available and well know to the skilled person.

In another embodiment, one or more (e.g. 1, 2, 3, 4, 5, 6, 7, 8, 9 or 10) of the L-amino acids are substituted with a D amino acid. In another embodiment, one or more (e.g. 1, 2, 3, 4, 5, 6, 7, 8, 9 or 10) N-methylated residues are included in the peptide. The peptide inhibitor/s of the invention can comprise, e.g., L-amino acids, D- amino acids, other non-natural amino acids, or combinations thereof.

Active variants include molecules comprising various tags at the N-terminus or the C-terminus of the peptide (e.g. tags comprising a stretch of heterologous amino acids). For example, the peptide inhibitor/s of the invention can comprise as tags at its N-terminus and/or at its C-terminus: 1, 2, 3, 4, 5, 6, 7, 8, 9 or 10 or more Lysine residues; 1, 2, 3, 4, 5, 6, 7, 8, 9 or 10 or more Arginine residues; 1, 2, 3, 4, 5, 6, 7, 8, 9 or 10 or more Glutamate residues; 1, 2, 3, 4, 5, 6, 7, 8, 9 or 10 or more Aspartate residues; combinations of these amino acid residues; or other polar tags that will be evident to a skilled worker. Other active variants include mutations of the Tau and/or Ab sequence which increase affinity of the inhibitory peptides for Tau and/or Ab.

In one embodiment of the invention, the peptide inhibitor/s of the invention is isolated or purified, using conventional techniques such as the methods described herein. By "isolated" is meant separated from components with which it is normally associated, e.g., components present after the peptide is synthesized. An isolated peptide can be a cleavage product of a protein which contains the peptide sequence. A "purified" inhibitory peptide can be, e.g., greater than 90%, 95%, 98% or 99% pure.

In one embodiment, to enhance the cell permeability of an inhibitory peptide of the invention, the peptide is fused to any of a variety of cell penetrating peptides (CPPs). CPPs typically have an amino acid composition that either contains a high relative abundance of positively charged amino acids such as lysine or arginine or has sequences that contain an alternating pattern of polar/charged amino acids and non- polar, hydrophobic amino acids. These two types of structures are referred to as polycationic or amphipathic, respectively. A third class of CPP's are the hydrophobic peptides, containing only apolar residues, with low net charge or have hydrophobic amino acid groups that are crucial for cellular uptake. Typical CPP's that can be fused to an inhibitory peptide of the invention are known such as, for example in WO 2018/005867, the contents of which are incorporated herein by reference.

Preferably, the peptide inhibitor/s of the invention can be synthesized (e.g., chemically or by recombinant expression in a suitable host cell) by any of a variety of art- recognized methods. In order to generate sufficient quantities of an inhibitory peptide for use in a method of the invention, a practitioner can, for example, using conventional techniques, generate nucleic acid (e.g., DNA) encoding the peptide and insert it into an expression vector, in which the sequence is under the control of an expression control sequence such as a promoter or an enhancer, which can then direct the synthesis of the peptide. For example, one can (a) synthesize the DNA de novo, with suitable linkers at the ends to clone it into the vector; (b) clone the entire DNA sequence into the vector; or (c) starting with overlapping oligonucleotides, join them by conventional PCR-based gene synthesis methods and insert the resulting DNA into the vector. Suitable expression vectors (e.g., plasmid vectors, viral, including phage, vectors, artificial vectors, yeast vectors, eukaryotic vectors, etc.) will be evident to skilled workers, as will methods for making the vectors, inserting sequences of interest, expressing the proteins encoded by the nucleic acid, and isolating or purifying the expressed proteins.

Another aspect of the invention is a pharmaceutical composition comprising one or more of the peptide inhibitor/s of the invention and a pharmaceutically acceptable carrier. Optionally, the components of the pharmaceutical composition can be detectably labeled, e.g. with a radioactive or fluorescent label, or with a label, for example one that is suitable for detection by positron emission spectroscopy (PET) or magnetic resonance imaging (MRI). For example, peptides of the invention can be coupled to a detectable label selected from the group consisting of a radioactive label, a radio- opaque label, a fluorescent dye, a fluorescent protein, a colorimetric label, and the like. In some embodiments, the inhibitory peptide is present in an effective amount for the desired purpose. The compositions may contain preservatives and/or antimicrobial agents as well as pharmaceutically acceptable auxiliary substances as required to approximate physiological conditions, such as pH adjusting and buffering agents, tonicity adjusting agents, wetting agents, detergents and the like.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use. For example, "pharmaceutically acceptable salts" of a compound means salts that are pharmaceutically acceptable, as defined herein, and that possess the desired pharmacological activity of the parent compound.

Another aspect of the invention is a polynucleotide encoding the peptide inhibitor/s of the invention. In embodiments of the invention, the polynucleotide is operably linked to a regulatory control sequence (e.g., a promoter or an enhancer) to facilitate production of the encoded protein following introduction (e.g. by transfection) into a suitable cell. Other embodiments include a cell comprising the expression vector; and a method of making the peptide inhibitor/s of the invention comprising cultivating the cell and harvesting the peptide thus generated.

Another aspect of the invention is a kit for carrying out any of the methods described herein.

One aspect of the invention is a method for reducing or inhibiting Tau and/or Ab aggregation, comprising contacting Tau and/or Ab proteins with an effective amount of one or more of the inhibitory peptides of the invention. Such a method can be carried out in solution or in a cell (e.g. cells in culture or in a subject).

Another aspect of the invention is a method for treating a subject having a disease or condition which is mediated by the presence of fibrillated Tau (sometimes referred to herein as a Tauopathy or a Tau-mediated disease or condition), comprising administering to the subject an effective amount of an inhibitory peptide or pharmaceutical composition of the invention. Among such diseases or conditions are, e.g., Alzheimer's disease. Another aspect of the invention is a method to prevent the onset of such diseases or conditions (e.g., Alzheimer's disease), or to treat a subject in the early stages of such diseases or conditions, or that is developing such a disease or condition, in order to prevent or inhibit development of the condition or disease.

An "effective amount" of the peptide inhibitor/s of the invention is an amount that can elicit a measurable amount of a desired outcome, e.g. inhibition of Tau and/or Ab aggregation or cytotoxicity; for a diagnostic assay, an amount that can detect a target of interest, such as an Tau and/or Ab aggregate; or in a method of treatment, an amount that can reduce or ameliorate, by a measurable amount, a symptom of the disease or condition that is being treated.

A "subject" can be any subject (patient) having aggregated (fibrillated) Tau and/or Ab molecules associated with a condition or disease which can be treated by a method of the present invention. In one embodiment of the invention, the subject has Alzheimer's disease. Typical subjects include vertebrates, such as mammals, including laboratory animals, dogs, cats, non-human primates and humans.

The peptide inhibitor/s of the invention can be formulated as pharmaceutical compositions in a variety of forms adapted to the chosen route of administration, for example, orally, nasally, intraperitoneally, or parenterally, by intravenous, intramuscular, topical or subcutaneous routes, or by injection into tissue.

Suitable oral forms for administering the inhibitors include lozenges, troches, tablets, capsules, effervescent tablets, orally disintegrating tablets, floating tablets designed to increase gastric retention times, buccal patches, and sublingual tablets.

The peptide inhibitor/s of the invention can be systemically administered, e.g., orally, in combination with a pharmaceutically acceptable vehicle such as an inert diluent or an assimilable edible carrier, or by inhalation or insufflation. They can be enclosed in coated or uncoated hard or soft shell gelatin capsules, can be compressed into tablets, or can be incorporated directly with the food of the patient's diet. For oral therapeutic administration, the compounds can be combined with one or more excipients and used in the form of ingestible tablets, buccal tablets, troches, capsules, elixirs, suspensions, syrups, wafers, and the like. For compositions suitable for administration to humans, the term "excipient" is meant to include, but is not limited to, those ingredients described in Remington: The Science and Practice of Pharmacy, Lippincott Williams & Wilkins, 21st ed. (2006) (hereinafter Remington's). The inhibitors can be combined with a fine inert powdered carrier and inhaled by the subject or insufflated. Such compositions and preparations should contain at least 0.1% compounds. The percentage of the compositions and preparations may, of course, be varied and may conveniently be between about 2% to about 60% of the weight of a given unit dosage form.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring can be added. When the unit dosage form is a capsule, it may contain, in addition to materials of the above type, a liquid carrier, such as a vegetable oil or a polyethylene glycol. A syrup or elixir may contain the active compound, sucrose or fructose as a sweetening agent, methyl and propylparabens as preservatives, a dye and flavoring such as cherry or orange flavor. Various other materials can be present as coatings or to otherwise modify the physical form of the solid unit dosage form. For instance, tablets, pills, or capsules can be coated with gelatin, wax, shellac or sugar and the like. Of course, any material used in preparing any unit dosage form should be pharmaceutically acceptable and substantially non-toxic in the amounts employed.

In addition, the peptide inhibitor/s of the invention can be incorporated into sustained-release preparations and devices. For example, the inhibitors can be incorporated into time release capsules, time release tablets, and time release pills. In some embodiments, the composition is administered using a dosage form selected from the group consisting of effervescent tablets, orally disintegrating tablets, floating tablets designed to increase gastric retention times, buccal patches, and sublingual tablets.

The peptide inhibitor/s of the invention may also be administered intravenously or intraperitoneally by infusion or injection. Solutions of the inhibitors can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations can contain a preservative to prevent the growth of microorganisms.

The pharmaceutical dosage forms suitable for injection or infusion can include sterile aqueous solutions or dispersions or sterile powders comprising the compounds which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants.

Sterile injectable solutions are prepared by incorporating the compounds in the required amount in the appropriate solvent with various of the other ingredients enumerated above, as required, followed by filter sterilization. In the case of sterile powders for the preparation of sterile injectable solutions, the preferred methods of preparation are vacuum drying and freeze drying techniques, which yield a powder of the active ingredient plus any additional desired ingredient present in the previously sterile-filtered solutions.

Useful solid carriers include finely divided solids such as talc, clay, microcrystalline cellulose, silica, alumina and the like. Other solid carriers include conventional nontoxic polymeric nanoparticles or microparticles. Useful liquid carriers include water, alcohols or glycols or water/alcohol/glycol blends, in which the compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Useful dosages of the peptide inhibitor/s of the invention or pharmaceutical compositions of the invention can be determined by comparing their in vitro activity, and in vivo activity in animal models. Methods for the extrapolation of effective dosages in mice, and other animals, to humans are known to the art.

Effective dosages and routes of administration of agents of the invention are conventional. The exact amount (effective dose) of the agent will vary from subject to subject, depending on, for example, the species, age, weight and general or clinical condition of the subject, the severity or mechanism of any disorder being treated, the particular agent or vehicle used, the method and scheduling of administration, and the like. A therapeutically effective dose can be determined empirically, by conventional procedures known to those of skill in the art. See, e.g., The Pharmacological Basis of Therapeutics, Goodman and Gilman, eds., Macmillan Publishing Co., New York. For example, an, effective dose can be estimated initially either in cell culture assays or in suitable animal models. The animal model may also be used to determine the appropriate concentration ranges and routes of administration. Such information can then be used to determine useful doses and routes for administration in humans. A therapeutic dose can also be selected by analogy to dosages for comparable therapeutic agents.

The particular mode of administration and the dosage regimen will be selected by the attending clinician, taking into account the particulars of the case (e.g., the subject, the disease, the disease state involved, and whether the treatment is prophylactic). Treatment may involve daily or multi-daily doses of compound(s) over a period of a few days to months, or even years.

The Examples below provide illustrative methods and materials that can be used in the practice the various embodiments of the invention disclosed herein.

### Examples

### Materials and Methods

### 1. Human brain samples

Hippocampal and frontal lateral cortex brain samples from sporadic Alzheimer's disease patients and control subjects were kindly provided by Dr. A. Rabano from Banco de Tejidos (Fundación CIEN, Instituto de Salud Carlos III, Madrid, Spain). Based on quantitative pathological features, the Alzheimer's brain specimens were classified according to Braak stages I (n=3), II (n = 6), III (n = 3), IV (n=1), V (n = 10) and VI (n = 8), and non-demented control subjects (n = 9). Written informed consent premortem was obtained from all patients.

### 2. Nomenclature

Classical nomenclature [2] for *MAPT* gene exon/intron numbers has been used consistently throughout the present invention, although some of the databases (such as ENSEMBL: https://www.ensembl.org/index.html) employ a different nomenclature assigning different numbers to exons and introns (according to ENSEMBL, intron retention would take place in intron 13).

As for the new Tau isoform described in this invention, the transcript including the translation of intron 12 up to the first stop codon has been named *TIR-MAPT* (*Truncated by Intron Retention* MAPT), while the protein generated from such transcript has been termed W-Tau, due to the appearance of two characteristic tryptophan residues (W) in this isoform, an amino acid that does not appear elsewhere within the Tau molecule.

In addition, Tau truncated by asparagine endopeptidase, as previously described by Zhang et al [63] is referred to as ET-Tau (*Endopeptidase Truncated Tau*) throughout the text.

### 3. Cell culture

HEK293T (CRL-11268, ATCC), SK-N-MC (HTB-10, ATCC) and SH-SY5Y (CRL-2266, ATCC) cells were cultured in DMEM or MEM supplemented with 10% fetal bovine serum, 2 mM glutamine, non-essential amino acids, 10 U/ml penicillin and 10 µg/ml streptomycin, at 37º C and 5% CO₂.

### 4. TIR-MAPT and ET-MAPT cloning

*TIR-MAPT* transcript was obtained from an SH-SH5Y RNA extract, using specific oligos (TauNt and TauD, see **table 1**) and was cloned into pBlueScript-SK+ (212205, Agilent Technologies) thanks to a TA-cloning strategy [37]. Using specific oligos that include the appropriate restriction sites (A1 and TIR-T-BglII and ET-T-BglII, see **table 1**) *TIR-MAPT* and *ET-MAPT* were subcloned into a eukaryotic expression vector pSG5 (216201, Agilent technologies). Similarly, Tau isoforms were cloned into a prokaryotic expression vector pRK172 using specific oligos (TAU-PRK172 fw, TIR-T-pRKpWPI rv and ET-T-pRKpWPI rv; see **table 1**). After cloning, all vectors were sequenced using the described oligos **(table 1)**.

**Table 1 Detailed information of the oligonucleotides used as primers for semi-quantitative, quantitative RT-PCR and cloning.**

| **Name** | **Sequence** |
|---|---|
| Oligos for semiquantitative RT-PCR | |
| TauA | ACCAGTTGACCTGAGCAAGG |
| TauB | GGTGGCCAGGTGGAAGTAAA |
| TauC | GACCAGCCTTTGTCCACTCA |
| TauD | AGCCAGTCAACACAGAAGCC |
| TauE | GACACCACTGGCGACTTGTA |
| TauG | CCATCATAAACCAGGAGGTGGC |
| TauH | GCGGCAGTGTGCAAATAGTC |
| GAPDH Fw | GAGAAGGCTGGGGCTCATTT |
| GAPDH Rv | AGTGATGGCATGGACTGTGG |

| Oligos for quantitative RT-PCR | |
|---|---|
| TIR-T-fw | CATAAACCAGGAGGTGGCCAG |
| TIR-T-rv | CACCCTACCCCCTTTACCTTTT |
| MAPT-E11-E13-fw | GTCGAAGATTGGGTCCCTGG |
| MAPT-E11-E13-rv | GACACCACTGGCGACTTGTA |

| Oligos for cloning | |
|---|---|
| ET-T-Pacl | GTTTAATTAATCAATTTCCTCCGCCAGGGACGTGGG |
| TIR-T-Pacl | AATTAATTAATGCCCATATACCCAAGGGCAGC |
| A22 | CAAGATCTCAATTTCCTCCGCCAGGGACGTGGG |
| Tau-Nt | ATGGCTGAGCCCCGCCAGGAG |
| TIR-T-BglII | GAGATCTTAATGCCCATATACCCAAGGGCAGC |
| ET-T-Pacl | GTTTAATTAATCAATTTCCTCCGCCAGGGACGTGGG |
| TIR-T-Pacl | AATTAATTAATGCCCATATACCCAAGGGCAGC |
| Tau-Nt | ATGGCTGAGCCCCGCCAGGAG |
| TIR-T-BglII | GAGATCTTAATGCCCATATACCCAAGGGCAGC |
| TAU-PRK172 fw | GCGGATCCATATGGCTGAGCCC |
| TIR-T-pRKpWPI rv | GCGAATTCTTAATGCCCATATACCCAAGGG |
| ET-T-pRKpWPI rv | GTTCTGAATTCTTAATTAATCAATTTCCTCCGCCAGG |

| Extra oligos for sequencing | |
|---|---|
| A22 | CAAGATCTCAATTTCCTCCGCCAGGGACGTGGG |
| A1 | GGCGAATTCGGATCCTATGGCTGAGCCCCGCCAGGAG |
| A4 | GCTGCTCCCCGCGGTGTG |
| Tau-Ct | ACCCTGCTTGGCCAGGGAGGC |
| Tau R1 Rv | CCGCTGTTGGAGTGCTCTTA |
| Tau 447 Rv | CGTTTTACCATCAGCCCCCT |
| pSGTau-fw | CTCACTATAGGGCGAATTCATG |
| pSGTau-rv | AGCGGAAGAGTCTAGAGTCG |

### 5. RNA-seq data analysis

RNA-seq raw data from 363 samples of 3 brain regions (frontal cortex, dorsolateral prefrontal cortex and hippocampus) of 180 human brain healthy donors were retrieved from the Genotype-Tissue Expression (GTEx) project [36] **(table 2).** SRA files were converted to FASTQ files and reads were re-mapped to human genome GRCh38 using STAR version 2.5.2a [18]. Gene expression quantification was performed with RSEM version 1.3.1 [35].

**Table 2. Number of healthy brain samples and donors per brain region within GTEx that were RNA-seq analyzed. Venn diagram shows number of individuals for each sample region.**

| **Brain Region** | **Number of samples** | **Duplicated samples** | **Triplicated samples** | **Number of donors** |
|---|---|---|---|---|
| Frontal cortex | 134 | 3 | 2 | 127 |
| Dorsolateral prefrontal cortex | 120 | 1 | 4 | 115 |
| Hippocampus | 109 | 3 | 1 | 100 |

The annotation file was retrieved from GENCODE (gencode.v23.annotation.gtf) and was modified in order to include a new *MAPT-*related gene (*TIR-MAPT*) whose genome coordinates are chr17:45894382-46018851, which includes part of the intron 12 (chr17: 46018731-46018851) as the 3' end of the gene in the region mapping the oligonucleotide sequence TauD **(Table 2** and **Fig. 1****).** Gene expression levels were obtained from RSEM as Transcripts Per Kilobase Million (TPM) values. Expression levels of *MAPT* and *TIR-MAPT* genes were analyzed per brain region. For those donors with more than one sample in the same brain region, analysis was done only in the sample with highest *MAPT* expression.

### 6. Bacteria culture and Tau purification.

Upon cloning, pRK172 vectors encoding different Tau isoforms were transformed in BL21 *E. coli* competent cells by electroporation, from which Tau was purified, as described elsewhere [49]. These bacteria were cultured in LB medium with 100 ng/ml of ampicillin at 37º C overnight. Bacterial suspensions were transferred to 1L of LB with 100 ng/ml of ampicillin and further incubated at 37º C, up until optical density readings at 600 nm ranged between 0.6 and 0.8. At this point, 0.4 mM of IPTG was added to each sample so as to trigger transcription, and incubated once again at 37ºC for 2 hours. The samples were centrifuged at 2,950 x g at 4º C for 20 minutes and pellets were resuspended in Tau buffer, which consists of buffer A (0.1M MES pH 6.4, 0.5 mM MgCl₂ and 2mM EGT) supplemented with 1 mM PMSF; 0.5 NaCl and 5 mM β-mercaptoethanol. Resuspended pellets were sonicated on ice at 24 microns peak to peak (mpp) 5 times for 1 minute, waiting 10 seconds between repetitions. Upon sonication, samples were centrifuged at 13,850 x g at 4º C for 10 minutes and the resultant supernatants were subjected to 5 minutes at 100º C and 5 minutes on ice before centrifuging one more time at 13,850 x g at 4º C for 30 minutes. Supernatants were kept and Tris 1M was added until pH reaches a level of 11, in order to get rid of residual DNA. Ammonium sulfate 50% was added to the samples and they were incubated agitating at 4º C for at least 1h before centrifuging again at 13,850 x g at 4º C for 1h. Pellets containing purified Tau were resuspended in buffer A and the purification process was checked by SDS-PAGE and Coomassie blue staining in order to check if any other protein bands appeared upon Coomassie blue staining.

### 7. Tau protein quantitation.

Protein concentration for each Tau isoform was firstly determined by Coomassie Blue staining comparison with known amounts of protein and compared to results obtained by ELISA (Abcam, ab273617). ELISA results yielded higher variability, especially for 3R Tau isoforms. Consequently, concentrations were calculated by using Coomassie Blue staining data adjusted by using absorbance measurements corrected by individually-calculated extinction coefficients (ε), based on the specific amino acid sequence of each isoform [24] **(Table 3)**. Namely, we measured Tau concentration as indicated by Kundel and collaborators [34]: absorbance at 280 nm (A₂₈₀) was measured for each Tau isoform and concentration was determined as the ratio between A280 and ε. Extinction coefficients can be estimated from the amino acid sequence of the protein [24], according to the equation: ε₂₈₀ = number of tryptophan residues (W) x 5500 + tyrosine residues x 1490 [17]. Using this equation and the amino acid composition of each Tau isoform, the extinction coefficients were calculated for each one **(Table 3)**. Calculated values forT42 and T30 coincide with those calculated by Kundel [34], which has been typically used for all CNS human Tau isoforms [21], but are not valid for W-Tau isoforms that contain 2 extra tryptophan residues nor for ET-Tau isoforms, which lack 1 tyrosine that appear on the rest of the isoforms.

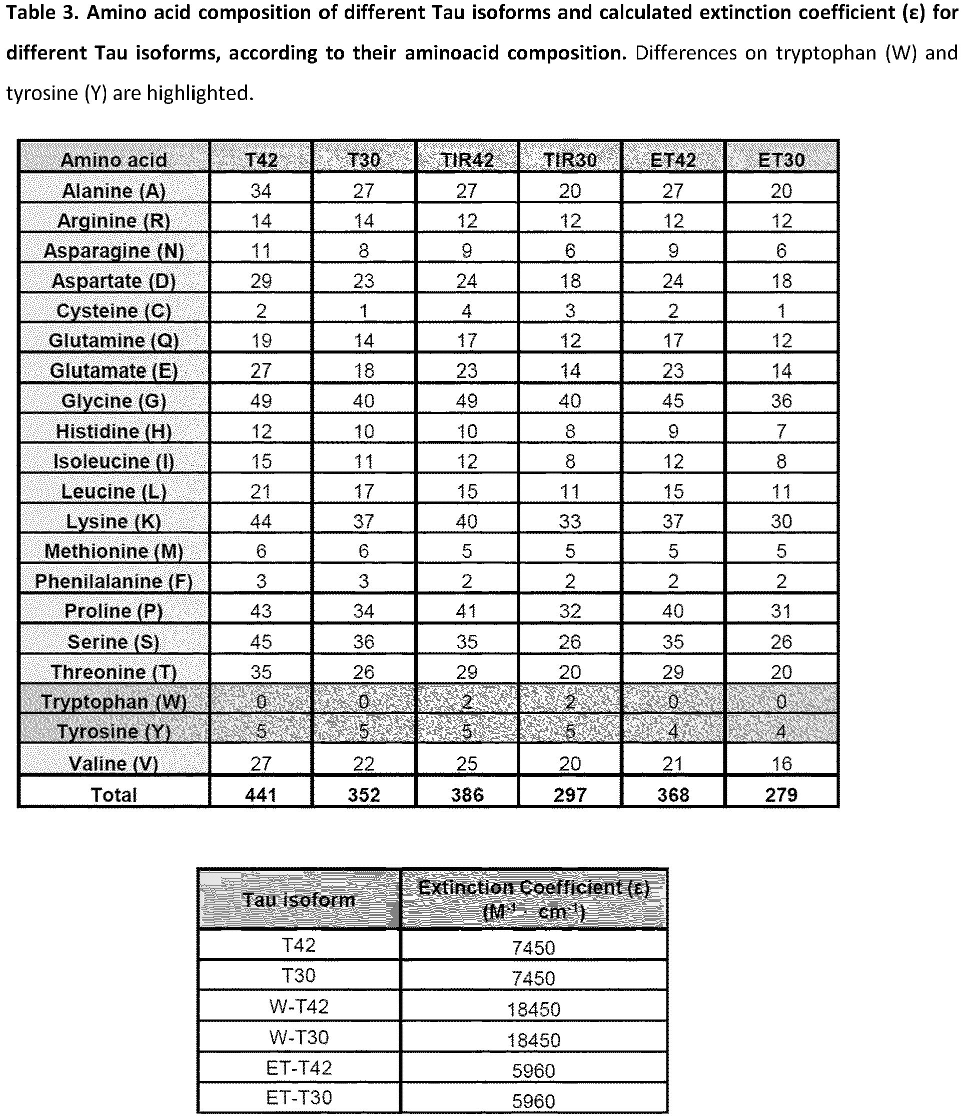

### 8. RNA extraction and purification

Either total RNA or cytosolic enriched fraction RNA was purified from cells using RNAeasy Mini Kit (74104, Qiagen) following the protocols described in Qiagen handbook. For brain tissue, previous homogenization using a TissueLyser (Retsch MM300, Qiagen, Hilden, Germany) (30 Hz, 5 min) with 5-mm stainless steel beads (69989, Qiagen) in 700 µl QIAzol Lysis Reagent (79306, Qiagen) was performed. RNA integrity numbers (RIN) were calculated using the Agilent 2100 Bioanalyzer system (Agilent Technologies), and only RNAs with RIN > 5 were used for RT-qPCR.

RNA was purified using RNAeasy Mini Kit (74104, Qiagen) with the following modifications. Cells were pelleted and frozen at -80º C. Pellets were carefully resuspended in 175 µl of precooled (4º C) buffer: 50 mM TrisHCl pH 8,140 mM NaCl, 1.5 mM MgCl₂, 0.5% (v/v) Nonidet P40 (1.06 g/ml) plus 1 mM DTT just before use and incubated on ice for 5 min. Lysates were centrifuged at 4º C for 2 min at 300 x g. Supernatants were kept as cytoplasmic enriched fraction. To each fraction, 600 µl of Buffer RLT were added. After vortexing, 430 µl of ethanol 100% were added to the homogenized lysate. Samples were transferred to RNeasy spin columns, centrifuged for 15s at 9,000 x g and the flow-through was discarded. Samples were treated with 10 µl of DNase in 70 µl of buffer RDD (RNase-free DNase Set 79254, Qiagen) for 15 min at room temperature. The rest of the extraction was performed following the protocol and RNA was collected in 30 µl of RNAse free water twice.

### 9. Semi-quantitative PCR

Total RNA was purified using RNAeasy Mini Kit (74104, Qiagen) following provider's guidelines. Cytoplasmic RNA fractions were purified as previously described. Retrotranscription was performed using 40 ng/µl of RNA with the Transcriptor First Strand cDNA Synthesis Kit (04379012001, Roche) using oligo(dT)18 primer. Semiquantitative PCR was performed using 1 µl of cDNA (0.5 ng/µl) supplemented with 2.5 mM MgCl₂, 0.2 mM each dNTP, 1.3 M betaine, 0.5 mM of each primer (described in **table 1**) and 0.025 U/ml of GoTaq^{®} Flexi DNA Polymerase (M829, Promega), in the following conditions: 95º C for 2 min, and 35 cycles (PCR1-8) or 30 cycles (PCR9-13) of 95º C for 45 s, 58.6º C for 45 s and 72º C for 45 s, followed by a final extension of 10 min at 72º C. PCR combinations are described in **table 4.**

**Table 4. Detailed information of the oligonucleotides used for each semi-quantitative RT-PCR and the length of the corresponding amplicons.**

| **PCR number** | **Forward primer** | **Reverse primer** | **Amplicon length** |
|---|---|---|---|
| 1 | TauB | TauC | 188 |
| 2 | TauB | TauD | 232 |
| 3 | TauG | TauC | 204 |
| 4 | TauG | TauD | 248 |
| 5 | TauA | TauC | 255 |
| 6 | TauA | TauD | 299 |
| 7 | TauH | TauC | 280 |
| 8 | TauH | TauD | 324 |
| 9 | TauA | TauE | 256 |
| 10 | TauB | TauE | 200 |
| 11 | TauG | TauE | 216 |
| 12 | TauH | TauE | 281 |
| 13 | GAPDH Fw | GAPDH Rv | 231 |

### 10. Detection of Tau levels by quantitative RT-PCR

RNA was retrotranscribed with the Transcriptor First Strand cDNA Synthesis Kit (04379012001, Roche) using 20 ng/µl RNA with oligo(dT)18 primer. Quantitative PCR was performed in a LightCycler480 (Roche) in the following conditions: 50º C for 2 min, 95º C for 10 min, and 40 cycles of 95º C for 15 s and 60º C for 1 min. Specific intron-spanning oligonucleotides against *MAPT* or *TIR-MAPT* transcript were designed (MAPT-E11-E13-fw/rv and TIR-T-fw/rv, respectively; see **table 1**)**.** Gene expression was normalized to GAPDH expression using TaqMan primer human GAPDH (Hs02758991_g1, Applied Biosystems). For every RT-qPCR experiment, only samples with RIN above 5 were used.

### 11. Detection of Tau levels by Western blot

Frozen brain tissue was homogenized using a TissueLyser (Retsch MM300, Qiagen, Hilden, Germany) (30 Hz, 5 min) with 5-mm stainless steel beads (69989, Qiagen) in total extraction buffer: 50 mM Tris-HCI pH 7.5, 300 mM NaCl, 0.5% SDS (sodium dodecyl sulphate) and 1% Triton X-100 (**Figures 2d****,** **2e** and **Fig. 13b**)**.** The same buffer was also used for cells (**Figures 2b****,** **2c****,** **3b****,** **4a****,** **5b** and **Fig. 11b** and **Fig. 12a****-b**)**.** For human cortex brain extracts, a strong lysis buffer (50 mM Tris-HCI pH 7.6, 400 mM NaCl, 1 mM EDTA, 1mM EGTA, 1% SDS) was also used in **Figure 7b****.** The homogenates are incubated for 15 min at 95º C, centrifuged (16,100 x g, 10 min) and the supernatant was considered the brain extract. Protein concentration was measured using the DC protein assay kit (500-0111, Bio-Rad). From each sample, equal amount of total protein was resolved on a 10% Bis-Tris gel and transferred to nitrocellulose membranes. Blots are probed with the corresponding primary antibodies (see below), followed by horseradish peroxidase-conjugated anti-mouse or anti-rabbit antibody (Dako, Glostrup, Denmark). Protein expression was quantified by measuring the ECL signal per lane with ImageJ software (http://rsbweb.nih.gov/ij/).

### 12. Antibodies.

Different antibodies were used for the different essays, as indicated in each one. Altogether, Tau 12 (Millipore, MAB2241; amino acids 6-18, diluted 1/1000), Tau 5 (Calbiochem, D00139295, amino acids 210-241, diluted 1/1000), Tau antibody (NOVUSBIO, NB100-82247, epitope around amino acid 231, diluted 1/500) and Tau 7.51 ([44], amino acids 315-376, diluted 1/100) were used as antibodies recognizing all Tau isoforms; Tau 1 (Chemicon, MAB3420, diluted 1/1000) recognizing dephosphorylated residues Ser195, 198, 199 and 202 and AT8 (phospho-Ser202/Thr205; Innogenetics, MN1020, diluted 1/100), AT180 (phospho-Thr231; Innogenetics, MN1040, diluted 1/100), Tau 404 (phospho-Ser404; Life Technologies, 44758G, diluted 1/1000), Tau 396 (phospho-Ser396; Life Technologies, 44752G, diluted 1/1000) and PHF1 (phospho-Ser396/Ser404, kind gift of Peter Davies [26], diluted 1/100) as antibodies recognizing phosphorylated Tau isoforms. In addition, β-actin (SIGMA A5441, diluted 1/20000) or GAPDH (Cell signalling, 2118, diluted 1/1000 antibodies were used as loading control.

A peptide composed of the W-Tau unique sequence (KKVKGVGWVGCCPWVYGH) (W-Tau peptide) was synthesised and a polyclonal IgG antibody against that peptide was obtained from Abyntek (Bizkaia, Spain), using New Zealand rabbit as host strain. This antibody was named W-Tau antibody and its specificity was tested (diluted 1/1000) to ensure it reacts exclusively with TIR-Tau but not with other Tau isoforms **(****Fig. 2a****-c).**

Immunoprecipitation was performed with W-Tau and total Tau (NOVUSBIO, NB100-82247) antibodies using SantaCruz Immunoprecipitation Kit (SC-2003) using 500 µg of whole cell extracts and following provider's guidelines.

### 13. Tau phosphorylation determination

HEK293T cells were transfected with pSG5 plasmids (2 µg/p60) encoding different Tau isoforms using Lipofectamine and Plus reagents following instructions of the supplier (#18324 and #11514, respectively, Life Technologies). 48 h after transfection, cells were collected, pelleted and homogenized in 200 µl of total extraction buffer. Protein concentrations were measured by using the DC protein assay kit (500-0111, Bio-Rad) and samples containing equivalent amounts of protein were analyzed by Western blot, as previously described, using nitrocellulose membranes. Immunodetection was performed with antibodies Tau 7.51 and Tau 5 for total Tau, Tau 1 for dephosphorylated Tau and AT8, AT180, Tau 404, Tau 396 and PHF1 for phosphorylated Tau.

### 14. Tau solubility determination

Once again, HEK293T cells were transfected with pSG5 plasmids (2 µg/p60) encoding different Tau isoforms using Lipofectamine and Plus reagents (Life Technologies). After 48 h, cells were collected, pelleted and homogenized in 150 µl of lysis buffer (50 mM Tris-HCI (pH 7,4), 150 mM NaCl, 20 mM NaF, 1 mM Na₃VO₄, 0.5 mM MgSO₄; supplemented with protease inhibitors cocktail, 04693159001 Roche) at 4ºC. Homogenates were centrifuged at 27,000 x g for 20 min at 4ºC. Pellets were discarded and 1% sarkosyl was added to the supernatants, being incubated for 1.5 h agitating at room temperature. Upon incubation, samples were centrifuged at 150,000 x g during 45 minutes at 4º C. Supernatants were kept as sarkosyl-soluble fraction and pellets were resuspended in 100 µl of a mix of total extraction buffer and loading buffer (1:1) and considered sarkosyl-insoluble fraction.

Results were further confirmed by carrying out a similar protocol to measure Triton X-100 solubility. Namely, pelleted cells were homogenized in 500 µl of lysis buffer (1% Triton X-100, 50 mM Tris-HCI pH 7, 100 mM NaCl and 1mM EDTA) and incubated 20 minutes at 4º C. The samples were centrifuged at 18,000 x g for 5 minutes and the supernatants were kept as soluble fraction. Pellets were resuspended in total extraction buffer (see point 11 of methods) and considered Triton-insoluble fraction.

In both cases, equivalent volumes of each sample were then analyzed by Western blotting.

### 15. Tau in vitro aggregation determination

Tau aggregates were grown for the different Tau isoforms by vapor diffusion in hanging drops in the standard way used for protein crystallizations [15]. The different Tau isoforms were purified from bacteria as described above and their concentration was estimated by measuring absorbance at 280 nm and taking into account individually-calculated extinction coefficients for each isoform **(table 3).** Equivalent quantities of each isoform were added to the corresponding volume of buffer A (0.1M MES pH 6.4, 0.5 mM MgCl₂ and 2 mM EGT) plus 50 mM NaCl in the presence of heparin, reaching a concentration of 1 mg/ml. The reservoir contained 0.2 M NaCl in buffer A. Aggregates were obtained after incubation for 10 days at room temperature (see also [49]). An aliquot was kept for electron microscopy analysis, and the rest of the samples were centrifuged in Airfuga at 28 pounds per square inch (psi) for 30 minutes at room temperature to separate soluble and aggregated protein. The fractionated proteins were characterized by electrophoresis and Western blot.

### 16. Tau microtubule binding capacity assay

For microtubule preparation, the brains of twelve 2-month-old C57BL/6J mice were homogenized with a potter in isotonic buffer (0.32 M sucrose, 1 mM EGTA, 1 mM MgCl₂, 10 mM phosphate buffer pH 7 and 1mM PMSF) at 4ºC. Supernatant was collected after 40 min ultracentrifugation at 100,000 x g at 4ºC in an Optima L-100 XP Ultracentrifuge (Beckman Coulter). 100 µl of this supernatant were incubated with equal quantity of protein for the different Tau isoforms, purified from bacteria as mentioned above during 30 minutes at 37ºC, in the presence of 30% glycerol, 1 mM PMSF and 1 mM GTP to promote tubulin polymerization. After that, samples were ultracentrifuged for 60 min at 100,000 x g at 25° C. Equivalent volumes of pellets containing microtubules and microtubule-bound Tau were analyzed by Western Blot, using Tau 5 and tubulin antibodies and the ratio between Tau 5 and tubulin signal was calculated for each isoform as their microtubule binding ratio, normalized to the corresponding full-length isoform.

### 17. GSK3 modulation

SK-N-MC human neuroblastoma cells (ATCC^{®} HTB-10^{™}) were treated with GSK3 inhibitors SB216763 (25 µM, GlaxoSmithKline compounds[13]) and AR-14418 (10 µM, AstraZeneca compound[7]) or Aβ (5 µg/ml, Neosystem Laboratoire, Strasbourg, France) for 24 h and whole cell extracts were used for Western blot analysis, probing the blots with W-Tau antibody. From those cells treated with AR-14418 and Aβ, RNA-enriched cytoplasmic fraction was also retrieved and *TIR-MAPT* RNA levels were assessed by quantitative RT-PCR as previously described.

### 18. Splicing factor binding site prediction analysis.

Exonic and intronic human *MAPT* sequences were retrieved from ENSEMBL (ENSG00000186868). Exon 12- exon 13 and exon 12 - intron 12 sequences (Exon 13 - exon 14 and exon 13 - intron 13, according to ENSEMBL nomenclature of Tau exons/introns) were analyzed using ESEFinder 3.0 (http://exon.cshl.edu/ESE/) [53]. ESEFinder settings were adjusted to show binding sites with score 4.0 or higher. The analysis was completed by a detailed bibliographic search comparing SRSF2 binding sites described in Masaki et al, 2019 and Cavaloc et al, 1999 [11, 38] with junctions *MAPT* Exon 12 - exon 13 and exon 12 - intron 12 sequences.

### 19. Statistical analysis

Quantitative data, represented as mean ± SD or SEM, were compared between groups using the two-tailed Student's t-test. For multiple comparisons, one-way ANOVA and Dunnett's test were performed to compare each isoform and the correspondent full length isoform. When the distribution of the data was not Gaussian a nonparametric Kruskal-Wallis test was used. The differences are given with their corresponding statistical significance or p value, which is the probability that the difference occurred merely by chance under the null hypothesis (* p≤0.05; ** p≤0.01; *** p ≤ 0.001; **** p ≤ 0.0001; N.S. not significant). The method used for each experiment is specified in the corresponding figure legend.

### Results

### New alternative splicing variant of MAPT present in mature human transcriptome

Recently, the presence of new protein isoforms generated by intron retention in neurodegenerative diseases such as Alzheimer's disease [45] and Huntington's disease [52] has been described. This, together with evidence of an asparagine-endopeptidase truncated Tau isoform [63], whose cleavage point coincides with the end of exon 12 led us to seek possible intron retention events within the intron between exons 12 and 13 (intron 12). Curiously, human intron 12 includes a canonical stop codon sequence followed by a polyadenylation canonical site, so intron 12-containing species would be translated as a truncated protein similar to the truncated isoform mediated by AEP (ET-Tau), with 18 more amino acids, which we have named W-Tau **(****Fig. 1a****).** Importantly, this isoform would be human-specific, since this polyadenylation site cannot be found in other species, such as mice. We could detect intron 12 retaining RNA species in which intron 11 was spliced-out in SH-SY5Y mature (polyA positive) total RNA and cytoplasmic enriched fraction **(****Fig. 1b****,** **Fig. 8** **and Tables 1 and 4).** This was observed by means of semi-quantitative PCR using an exon 11-matching forward oligo and intron 12 reverse oligos **(****Fig. 1a****-b, Table 4, PCR 5 and 6).** As expected, we could also detect the equivalent amplicon using an exon 12-matching reverse oligo in which intron 12 was spliced out **(****Fig. 1a****-b, Table 4, PCR 9).** Additionally, we could observe similar results using an exon 12 forward oligo and intron 12 reverse oligos **(****Fig 8a****-b, Table 4 PCR 1 and 2).** Genomic DNA contamination was ruled out using intron-spanning forward oligos **(****Fig. 8a****-b and Table 4, PCR 3, 4, 7 and 8).** Curiously, intron-spanning primers matching exon 10 to detect RNA species containing intron 12 never detected these as mature cytoplasmic polyA positive RNA **(****Fig. 8a****-b and Table 4, PCR 7 and 8).** Exon 10 is spliced-out in Tau isoforms with 3 repeats (Tau 3R) and it has been previously described that proliferative SH-SY5Y cells mainly express these Tau isoforms [57], so detection of RNA species with partial inclusion of exon 10 was not expected *a priori.* As a control, mature polyA positive RNA species including only exons were found in our samples (**Fig. 8a****-b** and **Table 4, PCR 10-12**), even detecting transcripts that include exon 10 (Tau 4R), although with decreased efficiency (**Fig. 8a****-b** and **table 4, PCR 12).** These results were also validated by qPCR using the same oligos **(****Fig. 8c****)** and different, qPCR-optimized ones **(****Fig. 1c****-d, Table 1).** Moreover, we were able to amplify whole-length *TIR-MAPT* cDNA from starting codon to intron 12 using specific primers (TauNt and TauD, **Table 1)** in SH-SY5Y cells. This cDNA would correspond to *TIR-MAPT* with 3 repeats and no inserts (TIR-T30).

More importantly, *TIR-MAPT* polyA positive mRNA was found in both hippocampus and frontal cortex in human samples **(****Fig. 1e****).** Remarkably, *TIR-MAPT* expression levels in human brain were more than 100-fold the expression found in SH-SY5Y, suggesting a higher relevance of this RNA species in human brains than in cultured cells.

Additionally, RNA-seq data from 363 samples from 3 different brain regions of 180 healthy donors were used to confirm the existence of *TIR-MAPT* transcripts **(Table 2).** *TIR-MAPT* gene mature mRNA containing the first part of intron 12 up until the canonical stop site was expressed in the majority of samples **(****Fig. 1f****)** supporting our previous findings in a big cohort of human samples. Gene expression of *MAPT* mature mRNA was compared with *TIR-MAPT* **(****Fig. 1g****).** Even though *TIR-MAPT* is expressed at lower levels, the great number of samples expressing this intron-retaining species suggests a relevant function in human brain. Among the RNA species containing intron 12, some of the reads would correspond to a putative Tau protein without inserts, and with 3 tandem repeats (T30), but substituting the 3'-terminal of the RNA for the sequence of intron 12 until the first stop codon; what we have named TIR-T30. The putative translation of this RNA species into a protein has been named W-T30, due to the presence of two tryptophan residues (W) in the sequence of intron 12 translation **(****Fig. 9****).** This RNA species would be equivalent to that found in SH-SY5Y cells.

### W-Tau expression in human brain.

With the aim of determining whether this new Tau isoform is present in human brain as a protein, we obtained a specific antibody against the unique 18 amino acid sequence of W-Tau peptide (KKVKGVGWVGCCPWVYGH; W-Tau antibody), corresponding to the translation of intron 12 until the canonical stop codon. To confirm the specificity of this antibody, we cloned *TIR-MAPT* cDNA obtained from SH-SY5Y (TIR-T30, which would correspond to W-T30 as a protein) into the expression vector pSG5 (216201, Agilent technologies) and used it to overexpress this isoform in HEK293T cells. We demonstrated that W-T30 could be specifically immunoprecipitated with both W-Tau (Abyntek) or Total Tau (NOVUSBIO, NB100-82247) antibodies **(****Fig 2a****).** and then that W-Tau antibody only precipitates W-Tau without any background on control untreated cells **(****Fig 2b****).** In both cases, the immunoprecipitated protein was confirmed to be Tau by detecting it by Western blot with Tau 7.51 antibody.

To further confirm this specificity and contrast it with otherTau isoforms, we further modified the eukaryotic vector pSG5 to include exon 10 corresponding to the fourth tandem repeat of the microtubule binding domain and the two Tau inserts (thus obtaining W-T42). Additionally, asparagine endopeptidase truncated Tau corresponding isoforms were also cloned into pSG5 expression vector (ET: Endopeptidase truncated; ET-T42 and ET-T30); mimicking the cut of endopeptidase by including a stop codon after the one that encodes asparagine 368. We used this vector encoding W-Tau, ET-Tau and the full-length equivalents (T42 and T30) to overexpress these proteins on HEK293T cells, together with control cells transfected with the empty pSG5 vector. We observed that, while total Tau antibodies (such as Tau 7.51) recognize all these isoforms, W-Tau antibody only shows signal for W-Tau isoforms, without any background on other species **(****Fig. 2c****).**

Once the specificity of the antibody was confirmed, we probed human hippocampal and frontal lateral cortex samples with it, confirming the existence of this novel Tau isoform as a protein in human brain **(****Fig 2d****-e).** In the first image, we show an example of a human subject that exhibits a predominant band close to 52 KDa that may correspond to a 4R form of W-Tau (**Fig 2d****,** 50 kDa band). Also, a smaller band at 38 KDa suggest the presence of W-T30 isoform in the hippocampal sample. Moreover, there is another band around 31 KDa that is recognized by W-Tau antibody, that might be explained as a proteolytic cleavage of the above bands, although further studies will be necessary to unravel the identity of this band. Then, we studied W-Tau expression in another 3 human subjects by comparing hippocampal and frontal lateral cortex samples **(****Fig. 2e****).** We could observe a predominant expression of 52 KDa 4R W-Tau bands, especially in frontal lateral cortex whose levels vary among individuals, and the 31 KDa band that may correspond to the truncated W-Tau form. All these results together indicate that intron 12 retaining Tau isoforms are expressed in human brain, of which two of them show similar molecular weights to those of W-T42 and W-T30 in cells upon overexpression.

### W-Tau phosphorylation

The pSG5 vectors encoding the different isoforms were employed to induce overexpression of these isoforms, in order to test W-Tau properties and compare them to those of full-length isoforms (T42 and T30) and those of asparagine endopeptidase truncated isoforms (ET42 and ET30) **(****Fig. 3a****).** Firstly, we studied the phosphorylation of W-Tau isoform, comparing it with the phosphorylation found in other Tau isoforms, expressed in HEK293T cells. We expected differential detections according to the epitopes that remain despite truncation and those that are lost because of it **(****Fig. 3a****).** Indeed, phosphorylation of AT8 sites Ser202/Thr205 and AT180 (phospho-Thr231) still occurred in truncated isoforms **(****Fig. 3b****-c).** However, as expected, PHF1 phosphorylation sites Ser396/Ser404 were not observed in truncated isoforms because these sequences are not present in those species. Also, some of the modifiable residues remained in dephosphorylated state, as shown by Tau 1 antibody label (dephospho- Ser195, 198, 199 and 202) **(****Fig 3b****-c)** and negative staining for phospho-Thr212 and phospho-Ser214 (AT100) (data not shown).

These results point out a similar post-translational processing of W-Tau when compared to other Tau isoforms, at least regarding its phosphorylation on the mentioned epitopes.

### W-Tau microtubule stabilization and binding capacity

Then, we analyzed the effect of W-Tau on the polymerization of mouse brain microtubules in *vitro* by means of electron microscopy in the presence or absence of W-Tau, showing a higher proportion of microtubules in the presence of W-Tau (W-T42) **(****Fig. 10a****).**

In addition, we analyzed the capacity of T42, W-T42 and ET-T42 purified from Tau-expressing bacteria **(****Fig. 10b****)** to bind to microtubules purified from mouse brain, separating microtubule-bound and free Tau by centrifugation and characterizing Tau enrichment with respect to tubulin in microtubule-bound fraction by Western blot with Tau 5 antibody. As expected, all the tested isoforms were able to bind to microtubules **(****Fig. 10b****-c),** since all of them contain microtubule-binding repeats. Of note, W-T42 showed a high affinity to bind microtubules compared to other isoforms harboring 4 repeats and 2 inserts **(****Fig. 10c****).** In accordance with previously published results [63], ET-T42 showed lower affinity to bind microtubules than full-length Tau.

### W-Tau aggregation capacity in cultured cells

We compared the aggregation tendency of different Tau isoforms when overexpressed in HEK293T cells, by quantification of Tau in Sarkosyl soluble versus insoluble protein fractions, with the insoluble fraction including Tau aggregates **(****Fig. 4a****).** With respect to full-length T42 aggregation capacity, we could observe that a similar soluble/aggregated protein ratio was found in full-length Tau isoforms. However, in truncated Tau isoforms, the ratio was different in W-Tau isoforms, with a higher proportion of soluble protein when compared to both full-length and ET-Tau isoforms, being significatively higher for W-T30 with respect to its correspondent full-length T-30 **(****Fig. 3b****-c).** These results were further confirmed by means of a similar assay that tested solubility with Triton X-100 **(****Fig. 11a****).** A similar analysis could be performed with the signal obtained from W-Tau antibody **(****Fig. 11b****-c).**

### In vitro aggregation of W-Tau

In addition, we carried out the analysis of the formation of protein aggregates by *in vitro* incubation of purified T42, W-T42 and ET-T42 with heparin, observing a reduced proportion of Tau aggregates when W-T42 was tested, compared to the rest of the isoforms **(****Fig. 4d****),** as determined by densitometry of electrophoretically fractionated protein bands present upon centrifugation in supernatant (non-aggregated protein) versus those present in pellet (aggregated protein) **(****Fig. 4e****).** These results support that W-Tau displays diminished aggregation tendency.

### Potential mechanism explaining intron 12 inclusion.

We performed an *in silico* study, along with a bibliographic search, of the potential splicing factors involved in the process. Results obtained with ESEFinder 3.0 indicate the presence of a high-scored SRSF6 splicing binding site in the canonical *MAPT* sequence, close to the beginning of Exon 13 **(****Fig. 12****).** This site is not present in the *TIR-MAPT* sequence*.* However, in its place, ESEFinder 3.0 indicates the appearance of a high-scored SRSF2 (also known as SC-35) splicing binding site in intron 12, in contrast to *MAPT* sequence splicing out intron 12 **(****Fig. 12****).** We re-analyzed *MAPT* exon 12 - exon 13 and exon 12 - intron 12 junction sequences using SRSF2 previously described binding sites [11, 38]. Surprisingly, we found that exon 12 - intron 12 junction sequence, AGGTAAAG, is extremely similar to a specific SRSF2 binding site AGGTRAG (R for any Purine, A or G) previously described by Masaki et al [38]. Both analyses suggested that SRSF2 may bind to *TIR-MAPT* sequence at the exon 12-intron 12 junction and/or at the beginning of intron 12 pointing to SRSF2 as a potential responsible for the regulation of this process. This evidence may be further supported by previous well-established evidence that SRSF2 is also involved in Tau exon 10 splicing [29], pointing out a regulatory function of this splicing factor on Tau alternative splicing that may be linked to the intron retention described in this work.

Additionally, previous work of our group suggests that the inhibition of GSK3-mediated SRSF2 phosphorylation increases the inclusion of exon 10 [29]. Thus, we tested the effect of the inhibition of SRSF2 phosphorylation carried out by the constitutively active GSK3 kinase [29] on the retention of *MAPT* intron 12. We detected increased *TIR-MAPT* cytoplasmic mature RNA levels in SK-N-MC cells in the presence of the GSK3β inhibitor AR-014418 [7], compared to both untreated and amyloid β treated cells **(****Fig. 5a****).** When we analyzed W-Tau protein levels, we could observe that SB-216763, other GSK3β inhibitor also results in increased W-Tau levels, although these increase is more relevant for AR-014418 **(****Fig. 5b****).** These preliminary data indicate that SRSF2 may be involved in this process via GSK3β regulation **(****Fig. 5c****).**

Finally, it is worth noting that the presence of beta amyloid peptide, that could stimulate GSK3 [28], did not result in a change in the level of neither *TIR-MAPT* nor W-Tau **(****Fig. 5a****-b),** probably because GSK3 is constitutively active and, therefore, amyloid treated cells behave as untreated control cells containing already SC35 in its phosphorylated form. However, as previously described, amyloid β did result in a significant increase of total *MAPT* RNA **(****Fig. 5a****).**

### Expression of TIR-MAPT RNA in the brain of AD patients.

We studied *TIR-MAPT* mRNA levels in a cohort of hippocampal brain samples from non-demented individuals and AD patients classified according to their Braak stage (see **Table 5).** The study of absolute levels of *TIR-MAPT* mRNA revealed that it slightly decreases in the first stages of the disease (Braak II and III) but increases in the advanced stages (Braak V and VI), although no significant differences were found **(****Fig. 6a****).** Total *MAPT* mRNA levels were similarly studied, finding in this case decreased expression in AD patients with respect to healthy individuals, more significantly in Braak stage VI **(****Fig. 6b****).** This data correlates with what we previously describe in the analysis of RNAseq dataset **(****Fig. 1g****).** Finally, when we studied *TIR-MAPT*/total-*MAPT* ratio we could observe a significant increase in Braak VI samples as well as a tendency towards an increased ratio in AD patients with respect to non-demented individuals **(****Fig. 6c****).**

**Table 5. Information of brain samples from control non-demented subjects and AD patients classified according to their Braak stage.**

| **Braak stage** | **Sex** | **Age** | **Hippo campal samples** | **Cortex samples** |
|---|---|---|---|---|
| Control | M | 41 | qPCR, WB | WB |
| Control | F | 58 | qPCR, WB | qPCR, WB |
| Control | F | 78 | qPCR | WB |
| Control | M | 43 | qPCR, WB | WB |
| Control | M | 56 | qPCR, WB | WB |
| Control | F | 49 | qPCR, WB | WB |
| Control | M | 84 | qPCR | WB |
| Control | M | 84 | | WB |
| Control | M | 78 | qPCR, WB | WB |
| Control | M | 14 | WB | |
| I | F | 87 | | WB |
| I | M | 77 | | WB |
| I | M | 78 | | WB |
| II | M | 87 | qPCR | WB |
| II | M | 80 | qPCR | WB |
| II | F | 85 | qPCR | WB |
| II | M | 84 | | WB |
| II | M | 103 | | WB |
| II | F | 82 | | WB |
| III | M | 85 | qPCR, WB | WB |
| III | M | 85 | qPCR, WB | WB |
| III | F | Not known | WB | |
| III | M | 81 | | WB |
| IV | F | 98 | | WB |
| V | F | 87 | | WB |
| V | F | 73 | qPCR, WB | WB I |
| V | F | 88 | qPCR, WB | WB |
| V | F | 82 | qPCR, WB | WB |
| V | M | 87 | qPCR, WB | WB |
| V | M | 81 | qPCR | WB |
| V | M | 62 | qPCR | WB |
| V | F | 89 | qPCR | WB |
| V | F | 80 | | WB |
| V | F | 84 | | WB |
| VI | M | 68 | | WB |
| VI | M | 76 | qPCR, WB | WB |
| VI | M | 88 | | WB |
| VI | F | 86 | | WB |
| VI | F | 81 | qPCR, WB | WB |
| VI | M | 92 | qPCR | WB |
| VI | F | 84 | qPCR, WB | WB |
| VI | F | 81 | qPCR, WB | WB |

### W-Tau presence in patients' brains

Finally, in accordance to what was found for control human brains **(****Fig. 2c** **and d),** we confirmed that W-Tau antibody was able to specifically immunoprecipitate W-Tau protein from human brain in extracts from AD patients (Braak stages V and VI) that could be identified as Tau again with a total-Tau antibody (Tau 7.51) **(****Fig. 7a****).**

Once this was confirmed, we analyzed the levels of W-Tau protein in the cortex of healthy individuals and AD patients classified according to their Braak stage **(****Fig. 7b****).** We could observe that W-Tau is expressed in both healthy and AD individuals. The analysis of both W-Tau bands showed a strong diminished expression in all Braak stages with respect to non-demented individuals **(****Fig 7c****).** Accordingly, the quantification of each band separately also yielded significant differences in all Braak stages with respect to non-demented individuals **(****Fig. 13a****).** On the other hand, the expression of total-Tau seems to increase at later stages of the disease, but we could not detect significant differences among groups when individuals are separated by Braak stages in our samples **(****Fig. 7c****).** However, the ratio of W-Tau with respect to total-Tau expression reveals an enrichment of W-Tau in advanced stages of the disease (Braak V and VI) **(****Fig. 7c****).**

Additionally, we could also obtain hippocampal samples of some of these individuals, and we studied W-Tau expression **(****Fig. 13b****).** In such conditions, similar to what we previously observed in **Fig. 2d** **and e**, we mainly detect 31 KDa (W-Tau truncated) and 50 KDa (W-T42) bands and, to a lesser extent, 38 KDa (W-t30) band. We could observe a trend towards diminished W-Tau expression in AD patients in the tree bands being significant in advanced stages of the disease for the 38 KDa (W-T30) band as well as in W-Tau/total Tau ratio **(****Fig. 13c** **and d)**

**These data together indicate an inverse correlation between W-Tau levels and disease progression that opposes total Tau levels behavior, strongly suggesting a relevant role of this new intron-retaining isoform in healthy individuals, whose loss would be related to the progression of the disease.** Taken RNA and protein data together **(****Fig. 6** and **Fig. 7****)** and considering the more marked presence of Tau aggregates in Total Tau but not in W-Tau, the changes on the amount of W-Tau in AD with respect to total Tau may be more related to differences on the protein turnover, rather than to their synthesis.

### References

1 Abascal F, Ezkurdia I, Rodriguez-Rivas J, Rodriguez JM, del Pozo A, Vázquez J, Valencia A, Tress ML (2015) Alternatively spliced homologous exons have ancient origins and are highly expressed at the protein level. PLoS computational biology 11:e1004325
2 Andreadis A (2005) Tau gene alternative splicing: expression patterns, regulation and modulation of function in normal brain and neurodegenerative diseases. Biochim Biophys Acta 1739: 91-103 Doi 10.1016/j.bbadis.2004.08.010
3 Andreadis A, Brown WM, Kosik KS (1992) Structure and novel exons of the human tau gene. Biochemistry 31: 10626-10633
4 Avale ME, Rodriguez-Martin T, Gallo JM (2013) Trans-splicing correction of tau isoform imbalance in a mouse model of tau mis-splicing. Hum Mol Genet 22: 2603-2611 Doi 10.1093/hmg/ddt108
5 Avila J, Lucas JJ, Perez M, Hernandez F (2004) Role of tau protein in both physiological and pathological conditions. Physiol Rev 84: 361-384 Doi 10.1152/physrev.00024.2003
6 Basurto-Islas G, Luna-Munoz J, Guillozet-Bongaarts AL, Binder LI, Mena R, Garcia-Sierra F (2008) Accumulation of aspartic acid421- and glutamic acid391-cleaved tau in neurofibrillary tangles correlates with progression in Alzheimer disease. J Neuropathol Exp Neurol 67: 470-483 Doi 10.1097/NEN.0b013e31817275c7
7 Bhat R, Xue Y, Berg S, Hellberg S, Ormö M, Nilsson Y, Radesäter AC, Jerning E, Markgren PO, Borgegård Tet al (2003) Structural insights and biological effects of glycogen synthase kinase 3-specific inhibitor AR-A014418. The Journal of biological chemistry 278: 45937-45945 Doi 10.1074/jbc.M306268200
8 Buée L, Bussière T, Buée-Scherrer V, Delacourte A, Hof PR (2000) Tau protein isoforms, phosphorylation and role in neurodegenerative disorders. Brain Research Reviews 33: 95-130
9 Caceres A, Kosik KS (1990) Inhibition of neurite polarity by tau antisense oligonucleotides in primary cerebellar neurons. Nature 343: 461-463 Doi 10.1038/343461a0
10 Caffrey TM, Joachim C, Paracchini S, Esiri MM, Wade-Martins R (2006) Haplotype-specific expression of exon 10 at the human MAPT locus. Human molecular genetics 15: 3529-3537
11 Cavaloc Y, BOURGEOIS CF, Kister L, Stévenin J (1999) The splicing factors 9G8 and SRp20 transactivate splicing through different and specific enhancers. Rna 5: 468-483
12 Chen K-L, Yuan R-Y, Hu C-J, Hsu CY (2010) Amyloid-β peptide alteration of tau exon-10 splicing via the GSK3p-SC35 pathway. Neurobiology of disease 40: 378-385
13 Coghlan MP, Culbert AA, Cross DA, Corcoran SL, Yates JW, Pearce NJ, Rausch OL, Murphy GJ, Carter PS, Cox LR (2000) Selective small molecule inhibitors of glycogen synthase kinase-3 modulate glycogen metabolism and gene transcription. Chemistry & biology 7: 793-803
14 Conrad C, Vianna C, Freeman M, Davies P (2002) A polymorphic gene nested within an intron of the tau gene: implications for Alzheimer's disease. Proc Natl Acad Sci U S A 99: 7751-7756 Doi 10.1073/pnas.112194599
15 Crowther R, Olesen 0, Smith M, Jakes R, Goedert M (1994) Assembly of Alzheimer - like filaments from full - length tau protein. FEBS letters 337: 135-138
16 D'Souza I, Poorkaj P, Hong M, Nochlin D, Lee VM, Bird TD, Schellenberg GD (1999) Missense and silent tau gene mutations cause frontotemporal dementia with parkinsonism-chromosome 17 type, by affecting multiple alternative RNA splicing regulatory elements. Proc Natl Acad Sci U S A 96: 5598-5603
17 Daniel BM, Edelstein S (1991) Protein methods. John Wiley and sons, City
18 Dobin A, Davis CA, Schlesinger F, Drenkow J, Zaleski C, Jha S, Batut P, Chaisson M, Gingeras TR (2013) STAR: ultrafast universal RNA-seq aligner. Bioinformatics 29: 15-21
19 Fernandez-Nogales M, Cabrera JR, Santos-Galindo M, Hoozemans JJ, Ferrer I, Rozemuller AJ, Hernandez F, Avila J, Lucas JJ (2014) Huntington's disease is a four-repeat tauopathy with tau nuclear rods. Nat Med 20: 881-885 Doi 10.1038/nm.3617
20 Fischer I, Baas PW (2020) Resurrecting the Mysteries of Big Tau. Trends in Neurosciences; 43(7):493-504 https://doi.org/10.1016/j.tins.2020.04.007.
21 Furukawa Y, Kaneko K, Nukina N (2011) Tau protein assembles into isoform-and disulfide-dependent polymorphic fibrils with distinct structural properties. Journal of Biological Chemistry 286: 27236-27246
22 Gamblin TC, Chen F, Zambrano A, Abraha A, Lagalwar S, Guillozet AL, Lu M, Fu Y, Garcia-Sierra F, LaPointe N et al (2003) Caspase cleavage of tau: linking amyloid and neurofibrillary tangles in Alzheimer's disease. Proc Natl Acad Sci U S A 100: 10032-10037 Doi 10.1073/pnas.1630428100
23 García-Escudero V, Gargini R, Martin-Maestro P, Garcia E, García-Escudero R, Avila J (2017) Tau mRNA 3' UTR-to-CDS ratio is increased in Alzheimer disease. Neuroscience letters 655: 101-108
24 Gill SC, Von Hippel PH (1989) Calculation of protein extinction coefficients from amino acid sequence data. Analytical biochemistry 182: 319-326
25 Goedert M, Crowther RA, Spillantini MG (1998) Tau mutations cause frontotemporal dementias. Neuron 21: 955-958
26 Greenberg SG, Davies P (1990) A preparation of Alzheimer paired helical filaments that displays distinct tau proteins by polyacrylamide gel electrophoresis. Proceedings of the National Academy of Sciences 87: 5827-5831
27 Griner SL, Seidler P, Bowler J, Murray KA, Yang TP, Sahay S, Sawaya MR, Cascio D, Rodriguez JA, Philipp S (2019) Structure-based inhibitors of amyloid beta core suggest a common interface with tau. eLife 8:e46924. doi: 10.7554/eLife.46924.
28 Hernandez F, de Barreda EG, Fuster-Matanzo A, Lucas JJ, Avila J (2010) GSK3: a possible link between beta amyloid peptide and tau protein. Experimental neurology 223: 322-325
29 Hernandez F, Perez M, Lucas JJ, Mata AM, Bhat R, Avila J (2004) Glycogen synthase kinase-3 plays a crucial role in tau exon 10 splicing and intranuclear distribution of SC35. Implications for Alzheimer's disease. The Journal of biological chemistry 279: 3801-3806 Doi 10.1074/jbc.M311512200
30 Himmler A (1989) Structure of the bovine tau gene: alternatively spliced transcripts generate a protein family. Mol Cell Biol 9: 1389-1396
31 Hinrich AJ, Jodelka FM, Chang JL, Brutman D, Bruno AM, Briggs CA, James BD, Stutzmann GE, Bennett DA, Miller SAet al (2016) Therapeutic correction of ApoER2 splicing in Alzheimer's disease mice using antisense oligonucleotides. EMBO Mol Med: 8: 328-345. Doi 10.15252/emmm.201505846
32 Hutton M, Lendon CL, Rizzu P, Baker M, Froelich S, Houlden H, Pickering-Brown S, Chakraverty S, Isaacs A, Grover Aet al (1998) Association of missense and 5'-splice-site mutations in tau with the inherited dementia FTDP-17. Nature 393: 702-705 Doi 10.1038/31508
33 Kim BM, Schultz LW, Raines RT (1999) Variants of ribonuclease inhibitor that resist oxidation. Protein science 8: 430-434
34 Kundel F, Hong L, Falcon B, McEwan WA, Michaels TC, Meisl G, Esteras N, Abramov AY, Knowles TJ, Goedert M (2018) Measurement of tau filament fragmentation provides insights into prion-like spreading. ACS chemical neuroscience 9: 1276-1282
35 Li B, Dewey CN (2011) RSEM: accurate transcript quantification from RNA-Seq data with or without a reference genome. BMC bioinformatics 12: 323
36 Lonsdale J, Thomas J, Salvatore M, Phillips R, Lo E, Shad S, Hasz R, Walters G, Garcia F, Young N (2013) The genotype-tissue expression (GTEx) project. Nature genetics 45: 580-585
37 Marchuk D, Drumm M, Saulino A, Collins FS (1991) Construction of T-vectors, a rapid and general system for direct cloning of unmodified PCR products. Nucleic Acids Res 19: 1154 Doi 10.1093/nar/19.5.1154
38 Masaki S, Ikeda S, Hata A, Shiozawa Y, Kon A, Ogawa S, Suzuki K, Hakuno F, Takahashi S-I, Kataoka N (2019) Myelodysplastic syndrome-associated SRSF2 mutations cause splicing changes by altering binding motif sequences. Frontiers in Genetics 10: 338
39 Miller SM, Moore MJ, Massey V, Williams Jr CH, Distefano MD, Ballou DP, Walsh CT (1989) Evidence for the participation of Cys558 and Cys559 at the active site of mercuric reductase. Biochemistry 28: 1194-1205
40 Moon H, Cho S, Loh TJ, Jang HN, Liu Y, Choi N, Oh J, Ha J, Zhou J, Cho S (2017) SRSF2 directly inhibits intron splicing to suppresses cassette exon inclusion. BMB reports 50:423
41 Motoi Y, Sahara N, Kambe T, Hattori N (2010) Tau and neurodegenerative disorders. Biomol Concepts 1: 131-145 Doi 10.1515/bmc.2010.017
42 Neve RL, Harris P, Kosik KS, Kurnit DM, Donlon TA (1986) Identification of cDNA clones for the human microtubule-associated protein tau and chromosomal localization of the genes for tau and microtubule-associated protein 2. Brain Res 387: 271-280
43 Novak M (1994) Truncated tau protein as a new marker for Alzheimer's disease. Acta virologica 38: 173-189
44 Novak M, Jakes R, Edwards PC, Milstein C, Wischik CM (1991) Difference between the tau protein of Alzheimer paired helical filament core and normal tau revealed by epitope analysis of monoclonal antibodies 423 and 7.51. Proc Natl Acad Sci U S A 88: 5837-5841
45 Ong C-T, Adusumalli S (2020) Increased intron retention is linked to Alzheimer's disease. Neural Regeneration Research 15: 259-260 Doi 10.4103/1673-5374.265549
46 Ozcelik S, Sprenger F, Skachokova Z, Fraser G, Abramowski D, Clavaguera F, Probst A, Frank S, Muller M, Staufenbiel Met al (2016) Co-expression of truncated and full-length tau induces severe neurotoxicity. Mol Psychiatry 21(12): 1790-1798. Doi 10.1038/mp.2015.228
47 Park C, Raines RT (2001) Adjacent cysteine residues as a redox switch. Protein Engineering 14: 939-942
48 Park SY, Ferreira A (2005) The generation of a 17 kDa neurotoxic fragment: an alternative mechanism by which tau mediates beta-amyloid-induced neurodegeneration. J Neurosci 25: 5365-5375 Doi 10.1523/JNEUROSCI.1125-05.2005
49 Perez M, Arrasate M, Montejo De Garcini E, Munoz V, Avila J (2001) In vitro assembly of tau protein: mapping the regions involved in filament formation. Biochemistry 40: 5983-5991
50 Polanco JC, Li C, Bodea L-G, Martinez-Marmol R, Meunier FA, Götz J (2018) Amyloid-β and tau complexity-towards improved biomarkers and targeted therapies. Nature Reviews Neurology 14: 22
51 Raj T, Li YI, Wong G, Humphrey J, Wang M, Ramdhani S, Wang Y-C, Ng B, Gupta I, Haroutunian V (2018) Integrative transcriptome analyses of the aging brain implicate altered splicing in Alzheimer's disease susceptibility. Nature genetics 50: 1584
52 Sathasivam K, Neueder A, Gipson TA, Landles C, Benjamin AC, Bondulich MK, Smith DL, Faull RL, Roos RA, Howland Det al (2013) Aberrant splicing of HTT generates the pathogenic exon 1 protein in Huntington disease. Proc Natl Acad Sci U S A 110: 2366-2370 Doi 10.1073/pnas.1221891110
53 Smith PJ, Zhang C, Wang J, Chew SL, Zhang MQ, Krainer AR (2006) An increased specificity score matrix for the prediction of SF2/ASF-specific exonic splicing enhancers. Human Molecular Genetics 15: 2490-2508 Doi 10.1093/hmg/ddl171
54 Trabzuni D, Wray S, Vandrovcova J, Ramasamy A, Walker R, Smith C, Luk C, Gibbs JR, Dillman A, Hernandez DG (2012) MAPT expression and splicing is differentially regulated by brain region: relation to genotype and implication for tauopathies. Human molecular genetics 21: 4094-4103
55 Tress ML, Abascal F, Valencia A (2017) Alternative splicing may not be the key to proteome complexity. Trends in biochemical sciences 42: 98-110
56 Tuerde D, Kimura T, Miyasaka T, Furusawa K, Shimozawa A, Hasegawa M, Ando K, Hisanaga S-i (2018) Isoform-independent and-dependent phosphorylation of microtubule-associated protein tau in mouse brain during postnatal development. Journal of Biological Chemistry 293: 1781-1793
57 Uberti D, Rizzini C, Spano PF, Memo M (1997) Characterization of tau proteins in human neuroblastoma SH-SY5Y cell line. Neurosci Lett 235: 149-153 Doi S0304394097007155
58 Valenca GT, Srivastava GP, Oliveira-Filho J, White CC, Yu L, Schneider JA, Buchman AS, Shulman JM, Bennett DA, De Jager PL (2016) The role of MAPT haplotype H2 and isoform 1N/4R in parkinsonism of older adults. PloS one 11: e0157452
59 Wang ET, Sandberg R, Luo S, Khrebtukova I, Zhang L, Mayr C, Kingsmore SF, Schroth GP, Burge CB (2008) Alternative isoform regulation in human tissue transcriptomes. Nature 456: 470
60 Wang Y, Mandelkow E (2016) Tau in physiology and pathology. Nat Rev Neurosci 17:5-21 Doi 10.1038/nrn.2015.1
61 Weingarten MD, Lockwood AH, Hwo SY, Kirschner MW (1975) A protein factor essential for microtubule assembly. Proc Natl Acad Sci U S A 72: 1858-1862
62 Zhang W, Tarutani A, Newell KL, Murzin AG, Matsubara T, Falcon B, Vidal R, Garringer HJ, Shi Y, Ikeuchi T (2020) Novel tau filament fold in corticobasal degeneration. Nature 580:283-287
63 Zhang Z, Song M, Liu X, Kang SS, Kwon IS, Duong DM, Seyfried NT, Hu WT, Liu Z, Wang JZet al (2014) Cleavage of tau by asparagine endopeptidase mediates the neurofibrillary pathology in Alzheimer's disease. Nat Med 20: 1254-1262 Doi 10.1038/nm.3700
64 Zhao Y, Tseng IC, Heyser CJ, Rockenstein E, Mante M, Adame A, Zheng Q, Huang T, Wang X, Arslan PEet al (2015) Appoptosin-Mediated Caspase Cleavage of Tau Contributes to Progressive Supranuclear Palsy Pathogenesis. Neuron 87: 963-975 Doi 10.1016/j.neuron.2015.08.020
65 Zilka N, Filipcik P, Koson P, Fialova L, Skrabana R, Zilkova M, Rolkova G, Kontsekova E, Novak M (2006) Truncated tau from sporadic Alzheimer's disease suffices to drive neurofibrillary degeneration in vivo. FEBS letters 580: 3582-3588

## Claims

1. An isolated tau-isoform protein or peptide, or a fragment thereof, comprising at the C-terminus domain amino acid sequence WVGCCPW (SEQ ID NO 1) or amino acid sequence GVGWVG, wherein the C-terminus domain is understood as the 30 amino acids sited at the end of the amino acid chain of the protein or peptide.

2. The isolated protein or peptide of claim 1, wherein said protein or peptide consists of amino acid sequence WVGCCPW (SEQ ID NO 1) or amino acid sequence GVGWVG.

3. The tau-isoform protein or peptide of claim 1, wherein the protein or peptide comprises at the C-terminus domain amino acid sequence KKVKGVGWVGCCPWVYGH (SEQ ID NO 2), wherein preferably the C-terminus domain is understood as the 20 or 18 amino acids sited at the end of the amino acid chain of the protein or peptide.

4. The tau-isoform protein or peptide of claim 1, wherein the protein or peptide consists of amino acid sequence KKVKGVGWVGCCPWVYGH (SEQ ID NO 2).

5. The tau-isoform protein or peptide according to claim 3, wherein the protein or peptide comprises peptide sequence:

MAEPRQEFEVMEDHAGTYGLGDRKDQGGYTMHQDQEGDTDAGLKAEEAGIGDTPSLEDEAA
GHVTQARMVSKSKDGTGSDDKKAKGADGKTKIATPRGAAPPGQKGQANATRIPAKTPPAPKT
PPSSGEPPKSGDRSGYSSPGSPGTPGSRSRTPSLPTPPTREPKKVAVVRTPPKSPSSAKSRLQTAP
VPMPDLKNVKSKIGSTENLKHQPGGGKVQIVYKPVDLSKVTSKCGSLGNIHHKPGGGQVEVKSE
KLDFKDRVQSKIGSLDNITHVPGGGNKKVKGVGWVGCCPWVYGH (SEQ ID NO 14).

6. The tau-isoform protein or peptide according to claim 3, wherein the protein or peptide consists of SEQ ID NO 14.

7. The tau-isoform protein or peptide according to claim 2, wherein the tau-isoform comprises peptide sequence:

MAEPRQEFEVMEDHAGTYGLGDRKDQGGYTMHQDQEGDTDAGLKESPLQTPTEDGSEEPGS
ETSDAKSTPTAEDVTAPLVDEGAPGKQAAAQPHTEIPEGTTAEEAGIGDTPSLEDEAAGHVTQA
RMVSKSKDGTGSDDKKAKGADGKTKIATPRGAAPPGQKGQANATRIPAKTPPAPKTPPSSGEP
PKSGDRSGYSSPGSPGTPGSRSRTPSLPTPPTREPKKVAVVRTPPKSPSSAKSRLQTAPVPMPDLK
NVKSKIGSTENLKHQPGGGKVQIINKKLDLSNVQSKCGSKDNIKHVPGGGSVQIVYKPVDLSKVT
SKCGSLGNIHHKPGGGQVEVKSEKLDFKDRVQSKIGSLDNITHVPGGGNKKVKGVGWVGCCP
WVYGH (SEQ ID NO 15).

8. The tau-isoform protein or peptide according to claim 7, wherein the tau-isoform consists of SEQ ID NO 15.

9. A composition comprising one or a plurality of tau-isoform protein/s or peptide/s according to any of claims 1 to 8.

10. A composition comprising a polynucleotide encoding at least one tau-isoform protein or peptide according to any of claims 1 to 8.

11. The composition of claim 10, wherein the polynucleotide is disposed within a vector selected for its ability to express the protein or peptide in a mammalian cell, preferably in a human cell.

12. A composition as defined in claim 9 or as defined in claim 10, for use in a method of inhibiting the formation of Ab aggregates or tau aggregates in a subject in need thereof.

13. A composition as defined in claim 9 or as defined in claim 10, for use in a method of inhibiting the development or progression of Ab plaque formation in a subject in need thereof.

14. A composition as defined in claim 9 or as defined in claim 10, for use in a method of inhibiting the development or progression of a tauopathy in a subject in need thereof.

15. A composition as defined in claim 9 or as defined in claim 10, for use in a method of inhibiting the development or progression of Alzheimer's disease in a subject in need thereof.

16. A composition comprising an effective amount of the protein as defined in claim 9 or the polynucleotide as defined in claim 10, for use in a method of treatment of a subject having an amyloid disease **characterized by** aggregation of Tau protein.

17. A pharmaceutical composition comprising a composition as defined in claim 9 or as defined in claim 10, and a pharmaceutically acceptable carrier including a peptide stabilizing excipient.

18. The pharmaceutical composition of claim 17, wherein the peptide stabilizing excipient comprises a preservative, a tonicity adjusting agent, a detergent, a hydrogel, a viscosity adjusting agent, or a pH adjusting agent.

19. An *in vitro* use of the protein or peptide of any of claims 1 to 8, as a biomarker for the prognosis of an amyloid disease **characterized by** aggregation of Tau protein in a human subject.

20. An *in vitro* use of the protein or peptide of any of claims 1 to 8, as a biomarker for the prognosis of Alzheimer's disease in a human subject.
